(19) Europäisches Patentamt
European Patent Office
Office européen des brevets

(11) Publication number: **0 242 170 B1**

(12) **EUROPEAN PATENT SPECIFICATION**

(45) Date of publication of patent specification: **18.09.91**

(51) Int. Cl.$^5$: **C07D 307/88**, C07D 407/14, B41M 5/00

(21) Application number: **87303231.2**

(22) Date of filing: **13.04.87**

# (54) Divinyl compounds and chromogenic recording-material prepared by use thereof.

(30) Priority: **16.04.86 JP 88961/86**
**01.05.86 JP 102909/86**
**02.02.87 JP 23361/87**

(43) Date of publication of application:
**21.10.87 Bulletin 87/43**

(45) Publication of the grant of the patent:
**18.09.91 Bulletin 91/38**

(84) Designated Contracting States:
**BE CH DE FR GB IT LI**

(56) References cited:
**EP-A- 0 062 544**
**EP-A- 0 188 377**
**DE-A- 2 218 895**
**DE-A- 2 614 944**
**US-A- 4 020 056**

**CHEMICAL ABSTRACTS, vol. 102, no. 22, 03 June 1985, Columbus, OH (US); p. 684, no. 195269f, Columbus, Ohio, US; & JP - A - 60 27589**

(73) Proprietor: **YAMADA CHEMICAL CO., LTD.**
**1-1 Kamitoba-Kamichoshicho Minami-ku**
**Kyoto-shi Kyoto-fu(JP)**

(72) Inventor: **Kawai, Hajime**
**3-12-12 Osumigaoka Tanabe-cho**
**Tsuzuki-gun Kyoto-fu(JP)**
Inventor: **Fujino, Yoshiharu**
**3-14-23 Osumigaoka Tanabe-cho**
**Tsuzuki-gun Kyoto-fu(JP)**
Inventor: **Shimizu, Youji**
**54 Daigo-Kakiharacho Fushimi-ku**
**Kyoto-shi Kyoto-fu(JP)**
Inventor: **Nieda, Seiichi 205 Rumieru-Nishikyogoku**
**6 Nishikyogoku-Suehiro-cho**
**Ukyo-ku Kyoto-shi Kyoto-fu(JP)**
Inventor: **Gendai, Kazuhiko 6-1-1106 Mukaijima-Danchi**
**14-8 Mukaijima-Yotsuyaike**
**Fushimi-ku Kyoto-shi Kyoto-fu(JP)**
Inventor: **Tsunemitsu, Katsuhiko**
**30-3 Daigo-Takahatacho Fushimi-ku**
**Kyoto-shi Kyoto-fu(JP)**

**EP 0 242 170 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

**CHEMICAL ABSTRACTS, vol. 102, no. 26, 01 July 1985, Columbus, OH (US); p. 589, no. 229544c, Columbus, Ohio, US; & JP - A - 60 08364**

**CHEMICAL ABSTRACTS, vol. 107, no. 6, 10 August 1987, Columbus, OH (US); p. 663, no. 49640a, Columbus, Ohio, US; & JP - A - 61 202 883 (Cat. P, A)**

(74) Representative: **Woods, Geoffrey Corlett et al**
**J.A. KEMP & CO. 14 South Square Gray's Inn London WC1R 5EU(GB)**

## Description

The present invention relates to a divinyl compound suitable for use as a chromogenic agent in a recording material such as a pressure-sensitive recording paper, heat-sensitive recording paper or electroheat sensitive recording paper, to a process for the preparation of such a compound and to a recording material incorporating such a compound.

DE-A-2,218,895 describes a chromogenic tetrachlorophthalide of formula:

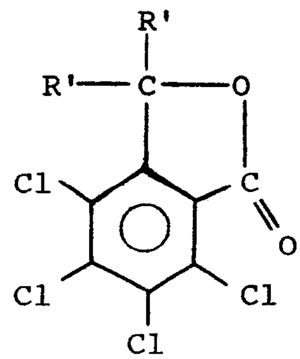

wherein each R' is an alkyl, phenyl, phenylalkyl or alkoxy substituted or unsubstituted p-aminophenyl, pyrrol-2-yl or indol-3-yl group, wherein the alkyl groups contain from 1 to 4 carbon atoms.

EP-A-62,544 describes a chromogenic phthalide derivative of formula:

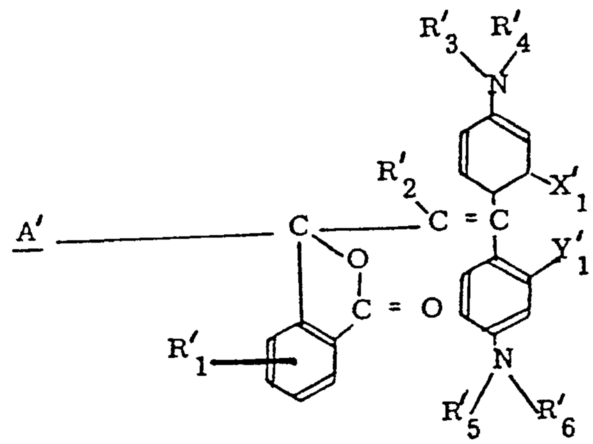

wherein $R'_1$ is hydrogen or at least one of halogen, alkyl, alkoxy, nitro, amino and substituted amino; $R'_2$ is hydrogen or alkyl;
each $R'_3$, $R'_4$, $R'_5$ and $R'_6$ is hydrogen, alkyl, aralkyl, substituted aralkyl, aryl or substituted aryl, or one or both of $R'_3$ and $R'_4$, together with the adjacent nitrogen, may form a heterocyclic ring and one or both of $R'_5$ and $R'_6$, together with the adjacent nitrogen, may form a heterocyclic ring;
each $X'_1$ and $Y'_1$ is hydrogen, alkyl or alkoxy; and A' may be various phenyl or diphenylvinyl groups.

Chemical Abstracts 102:195296f describes a chromogenic phthalide derivative of formula:

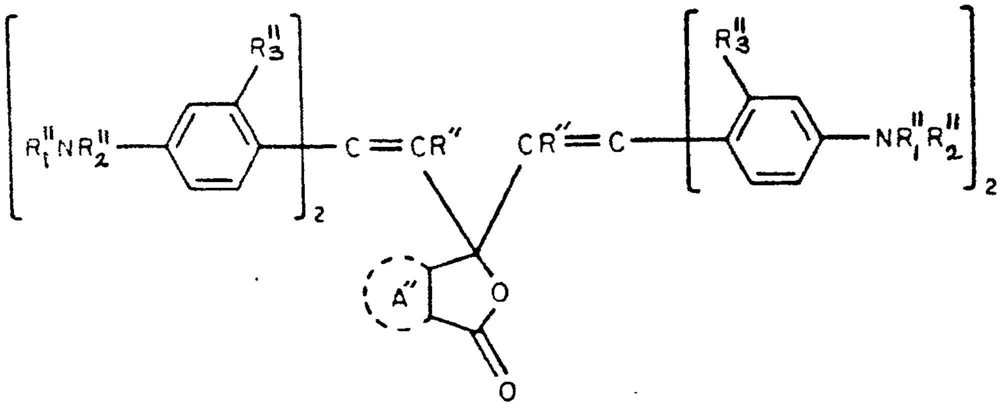

wherein:

R" is hydrogen or alkyl; $R_1$" and $R_2$" are alkyl, an aliphatic cyclic moiety, aryl or aralkyl or form heterocyclic rings with each cther or by bonding with the carbon atoms in the benzene ring; $R_3$" is hydrogen, alkyl, alkoxy or acyloxy; and A" is a benzene ring or a 6-membered heterocyclic aromatic ring containing 1 or 2 nitrogen atoms.

Chemical Abstracts 107:49640a discloses a pressure-sensitive recording paper comprising a combination of a FE(III) or a V compound and a compound of formula (I') or (II'):

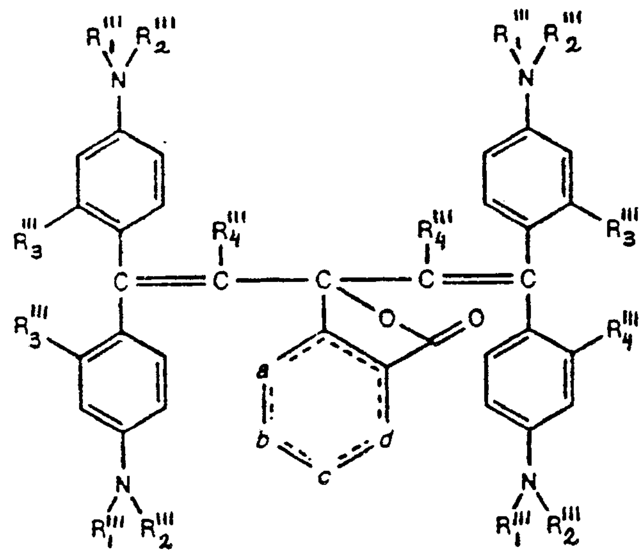

(I')

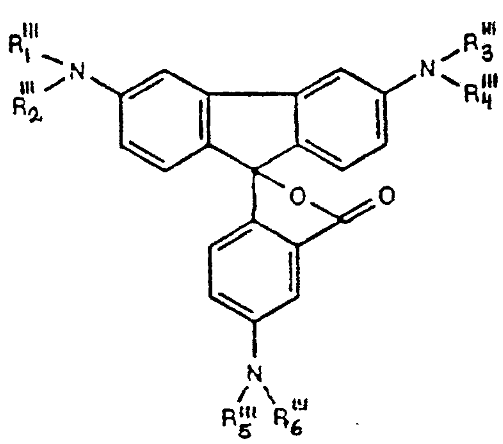

wherein, in formula (I'), $R_1$''' and $R_2$''' are alkyl or aryl or, together or in conjunction with an adjoining benzene ring, form a heterocycle; $R_3$''' is hydrogen, alkyl, alkoxy or aryloxy; $R_4$''' is hydrogen or alkyl; and a,b,c and d are carbon or nitrogen or a-b, b-c or c-d form another aromatic ring; and, in formula (II'), $R_{1-6}$'''

are $C_{1-4}$ alkyl.

Chemical Abstracts 102:229544c discloses a leuco pigment of formula (I") or (III"):

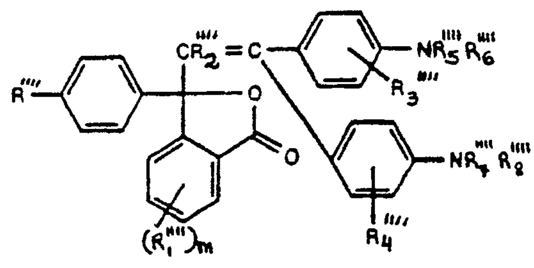

(I")

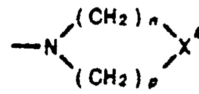

(II")

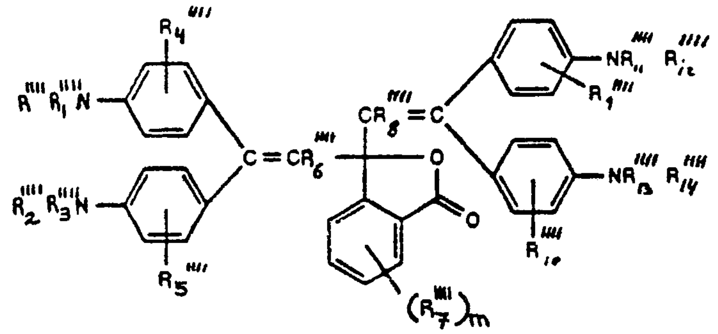

(III")

wherein, in formula (I"), R"" is hydrogen, halogen, alkyl, alkoxy, $NR_9$"" $R_{10}$"" or (II); $R_1$"" is hydrogen, halogen, alkyl or alkylamino; $R_2$"" is hydrogen or alkyl; $R_3$"" and $R_4$"" are hydrogen, alkyl or alkoxy; $R_{5-8}$"" are alkyl or cycloalkyl or $R_5$"" and $R_6$"" or $R_7$"" and $R_8$"" form a heterocycle with an adjacent N; m is 1 to 4, $R_9$"" and $R_{10}$"" are alkyl or cycloalkyl, n and p are at least one with the proviso that n + p is at least 4; and X"" is 5,0 or $CH_2$; and, in formula (III""), R"", $R_{1-3}$"" and $R_{11-14}$"" are alkyl or cycloalkyl or R"" and $R_1$"", $R_2$"" and $R_3$"", $R_{11}$"" and $R_{12}$"" or $R_{13}$"" and $R_{14}$"" form a heterocycle with an adjacent N; $R_4$"", $R_5$"", $R_9$"" and $R_{10}$"" are hydrogen, alkyl or alkoxy; $R_6$"" and $R_8$"" are hydrogen or alkyl; $R_7$"" is hydrogen, halogen, alkyl or alkylamino and m is 1 to 4.

The color image provided by the following black-coloring fluoran compound (A), which has been used as a conventional chromogenic agent for a recording-material, does not have any absorption in the near infrared region. Accordingly the color image cannot be read by an optical letter-reading apparatus (refer to Figure 2):

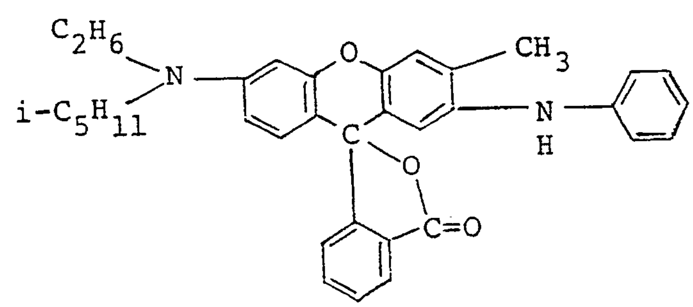

(A)

(refer to JP-B-56-52759/1981).

Chromogenic agents having an absorption in the near infrared region have been recently proposed in JP-B-58-5940/1983, JP-A-59-199757/1984 and JP-A-60-230890/1985. However, each of the proposed compounds has the following defects and a satisfactory chromogenic agent has not yet been obtained.

Compound (B) (see below) of JP-B-58-5940/1983 and compound (C) (see below) of JP-A-60-230890/1985 are themselves strongly colored yellow and besides spontaneously color. These defects have a very bad effect on the production of recording materials.

Although the fluorene compound (D) (see below) of JP-A-59-199757/1984 is colorless, its chromogenic property and the stability of color image produced are poor.

Furthermore, the hue of each of compounds (B), (C) and (D) when developed is green. Accordingly, to obtain a blackish color, another chromogenic agent giving a red or black color must be added in a large amount. Since the chromogenic property and the chromogenic speed of such an agent are different from those of compounds (B), (C) and (D) and, particularly, the light-resistance of red-chromogenic agents is generally poor, adverse influences such as unbalance of color-development and reduction of light-resistance cannot be avoided.

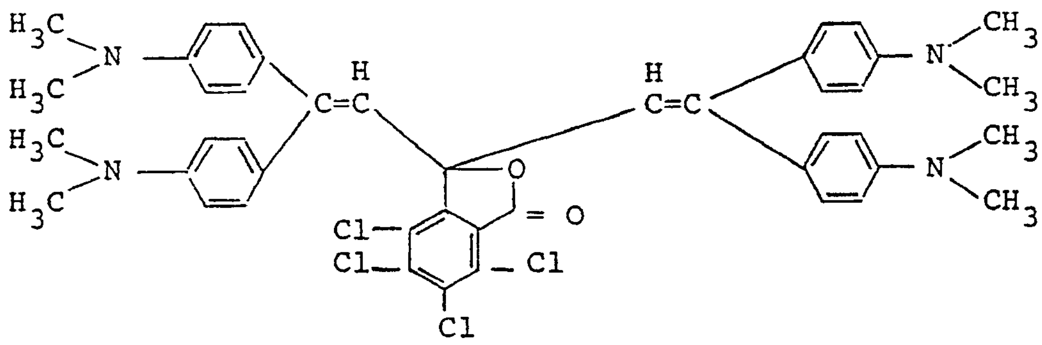

(B)

(refer to JP-B-58-5940/1983).

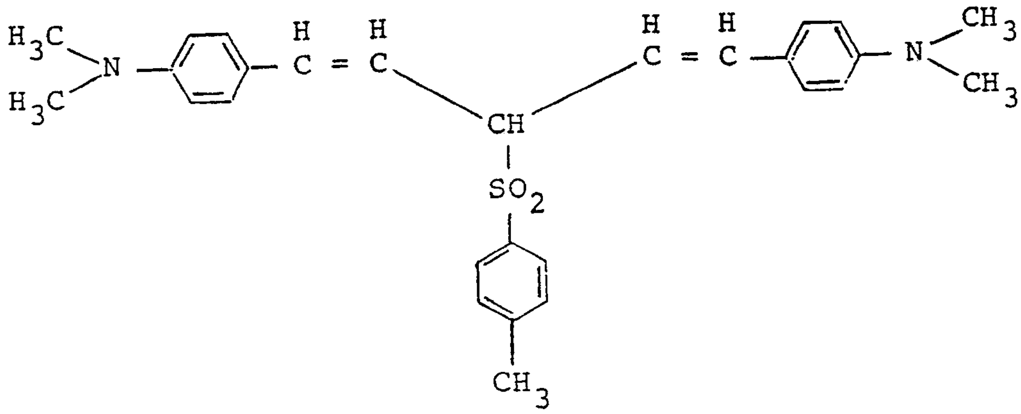

(C)

(refer to JP-A-60-230890/1985).

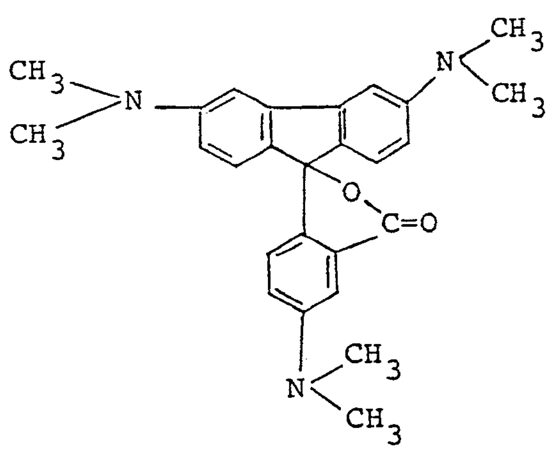

(D)

(refer to JP-A-59-199757/1984).

The present inventors have sought to improve the defects of the conventional chromogenic agents.

The present invention provides a divinyl compound represented by the formula (I):

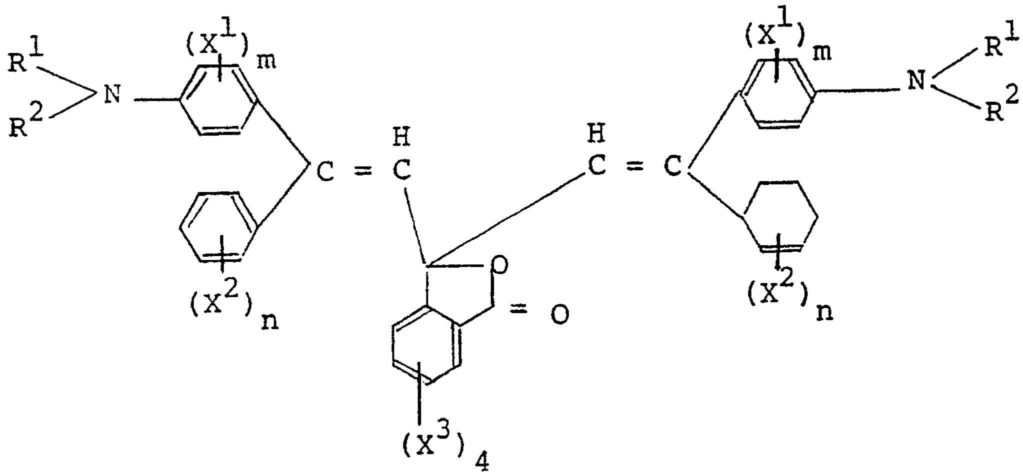

(I)

wherein $X^1$ represents an alkyl group, an alkoxy group or a halogen atom; $X^2$ represents an alkyl group, an alkoxy group, a halogen atom or $R^3O$-; $X^3$ represents a halogen atom; $R^1$ represents a hydrogen atom, an alkyl group, an alkoxyalkyl group, a haloalkyl group, an alkenyl group, an aralkyl group, a cyclohexylmethyl group, a methylcyclohexyl group, a trimethylcyclohexyl group, a furfuryl group, a tetrahydrofurfuryl group or a tetrahydropyrane-2-yl-methyl group; $R^2$ represents any group defined by $R^1$ above, a cycloalkyl group, a phenyl group, a benzyl group, a phenyl or a benzyl group substituted by chlorine atom(s), bromine atom(s) or alkyl group(s) which have not more than four carbon atoms or by an alkoxy group which has not more than four carbon atoms; $R^3$ represents a cycloalkyl group, an alkoxyalkyl group, an alkenyl group, a haloalkyl group or an aralkyl group; and m and n each represents 0, 1, 2 or 3; wherein each $X^1$ or each $X^2$ is the same or different when m or n respectively is 2 or 3 and each $X^3$ is the same or different. (Hereinafter the same sign means the same meaning).

The divinyl compounds represented by the formula (I) are by themselves almost colorless, extremely stable in the atmosphere, have no subliming property and spontaneously chromogenic property and dissolve extremely well in organic solvents. They give a blackish color rapidly with a developer and the color image produced has excellent light-resistance and moisture-resistance. Furthermore, since the color image has a strong absorption between 700 and 1000 nm in addition to the visible region, the color image has the distinctive feature that it is possible to be read by optical letter-reading apparatus using near infrared rays (such as OCR and OMR) and by barcode reading apparatus. Thus the divinyl compounds of the present invention (hereinafter sometimes referred to as the present compounds) are extremely valuable and can be used as chromogenic agents for ordinary recording-material requiring a black color, for which demand is rapidly increasing recently, as well as material readable by, for example, OCR or OMR.

Preferred compounds of the present invention are those wherein $R^1$ represents an alkyl group having not more than six carbon atoms, an alkoxyalkyl group having not more than six carbon atoms, a haloalkyl group having not more than six carbon atoms, an alkenyl group having not more than six carbon atoms, a cyclohexyl group, a cyclohexylmethyl group, a methylcyclohexyl group, a trimethylcyclohexyl group, a furfuryl group, a tetrahydrofurfuryl group, a tetrahydropyrane-2-yl-methyl group, a benzyl group, or a benzyl group substituted with an alkyl group having not more than four carbon atoms; $R^2$ represents any group defined by $R^1$ above, a phenyl group, a phenyl group substituted with a chlorine atom or an alkyl group having not more than four carbon atoms; $X^1$ represents a methyl group, a methoxy group or an ethoxy group; $X^2$ represents a halogen atom, an alkyl group having not more than six carbon atoms, an alkoxy group having not more than six carbon atoms, an alkoxyalkoxy group having not more than six carbon atoms, a cyclohexyloxy group, a benzyloxy group, a phenoxy group or a phenoxy or a benzyloxy group substituted with an alkyl or an alkoxy group both of which have not more than four carbon atoms; m is 0 or 1 and n is 0, 1 or 2.

More preferred compounds of the present invention are those wherein $R^1$ represents an alkyl group having not more than six carbon atoms; $R^2$ represents an alkyl group having not more than six carbon atoms or a cyclohexyl group; $X^2$ represents a halogen atom, an alkyl group having not more than six carbon atoms, an alkoxy group having not more than six carbon atoms, an alkoxyalkoxy group having not more than six carbon atoms, a cyclohexyloxy group, a phenoxy group, a benzyloxy group or a phenoxy or benzyloxy group substituted with an alkyl or an alkoxy group both of which have not more than four carbon

atoms; $X^3$ represents a chlorine atom, m is 0 and n is 0, 1 or 2.

Even more preferred compounds of the present invention are those wherein $R^1$ represents a methyl group or an ethyl group; $R^2$ represents a methyl group, an ethyl group, an n-propyl group or an iso-propyl group; $X^2$ represents a methyl group, an alkoxy group having not more than five carbon atoms and n is 1 or 2.

The divinyl compounds represented by the formula (I) have unexpectedly excellent properties such as solubility, the color of the compound itself, the hue of the developed color, the chromogenic property, the absorbancy of near infrared rays and the stability of the color image produced.

Of the attached drawings, Figures 1 and 2 are the reflection spectra of a color image of a pressure-sensitive and heat-sensitive recording paper incorporating the present compound and a comparison compound, respectively, and Figure 3 is the reflection spectra of a texture of a heat-sensitive recording paper incorporating the present compound and the comparison compound.

Examples of the present compounds are given below.

Every compound is an almost colorless or pale yellow solid and develops a blue-black to black color rapidly by the action of activated clay.

1. 3,3-Bis[2-(p-dimethylaminophenyl)-2-(p-methoxyphenyl)ethenyl]-4,5,6,7-tetrachlorophthalide
2. 3,3-Bis[2-(p-dimethylaminophenyl)-2-(p-ethoxyphenyl)ethenyl]-4,5,6,7-tetrachlorophthalide
3. 3,3-Bis(2-(p-diethylaminophenyl)-2-(p-methoxyphenyl)ethenyl]-4,5,6,7-tetrachlorophthalide
4. 3,3-Bis(2-(p-dimethylaminophenyl)-2-(p-propoxyphenyl)ethenyl]-4,5,6,7-tetrachlorophthalide
5. 3,3-Bis[2-(p-dimethylaminophenyl)-2-(p-isopropoxyphenyl)ethenyl]-4,5,6,7-tetrachlorophthalide
6. 3,3-Bis[2-(p-dimethylaminophenyl)-2-(p-butoxyphenyl)ethenyl]-4,5,6,7-tetrachlorophthalide
7. 3,3-Bis[2-(p-dimethylaminophenyl)-2-(p-isobutoxyphenyl)ethenyl]-4,5,6,7-tetrachlorophthalide
8. 3,3-Bis[2-(p-methylethylaminophenyl)-2-(p-methoxyphenyl)ethenyl]-4,5,6,7-tetrachlorophthalide
9. 3,3-Bis[2-(p-methylpropylaminophenyl)-2-(p-methoxyphenyl)ethenyl]-4,5,6,7-tetrachlorophthalide
10. 3,3-Bis[2-(p-dimethylaminophenyl)-2-(p-methylphenyl)ethenyl]-4,5,6,7-tetrachlorophthalide
11. 3,3-Bis[2-(p-ethylpropylaminophenyl)-2-(p-methoxyphenyl)ethenyl]-4,5,6,7-tetrachlorophthalide
12. 3,3-Bis[2-(p-methylisopropylaminophenyl)-2-(p-methoxyphenyl)ethenyl]-4,5,6,7-tetrachlorophthalide
13. 3,3-Bis[2-(p-ethylisopropylaminophenyl)-2-(p-methoxyphenyl)ethenyl]-4,5,6,7-tetrachlorophthalide
14. 3,3-Bis[2-(p-dipropylaminophenyl)-2-(p-methoxyphenyl)ethenyl]-4,5,6,7-tetrachlorophthalide
15. 3,3-Bis[2-(p-methylbutylaminophenyl)-2-(p-methoxyphenyl)ethenyl]-4,5,6,7-tetrachlorophthalide
16. 3,3-Bis[2-(p-ethylbutylaminophenyl)-2-(p-methoxyphenyl)ethenyl]-4,5,6,7-tetrachlorophthalide
17. 3,3-Bis[2-(p-diethylaminophenyl)-2-(p-ethoxyphenyl)ethenyl]-4,5,6,7-tetrachlorophthalide
18. 3,3-Bis[2-(p-dimethylaminophenyl)-2-(m-methyl-p-methoxyphenyl)ethenyl]-4,5,6,7-tetrachlorophthalide
19. 3,3-Bis[2-(p-dimethylaminophenyl)-2-(p-pentyloxyphenyl)ethenyl]-4,5,6,7-tetrachlorophthalide
20. 3,3-Bis[2-(p-dimethylaminophenyl)-2-(p-methoxyphenyl)ethenyl]-4,5,6,7-tetrabromophthalide
21. 3,3-Bis[2-(p-dimethylaminophenyl}-2-(p-isopentyloxyphenyl)ethenyl]-4,5,6,7-tetrachlorophthalide
22. 3,3-Bis[2-(p-dimethylaminophenyl)-2-(m,p-dimethoxyphenyl)ethenyl]-4,5,6,7-tetrachlorophthalide
23. 3,3-Bis[2-(p-methylpropylaminophenyl)-2-(p-ethoxyphenyl)ethenyl]-4,5,6,7-tetrachlorophthalide
24. 3,3-Bis[2-(p-ethylpropylaminophenyl)-2-(p-ethoxyphenyl)ethenyl]-4,5,6,7-tetrachlorophthalide
25. 3,3-Bis[2-(p-dimethylaminophenyl)-2-(p-ethylphenyl)ethenyl]-4,5,6,7-tetrachlorophthalide
26. 3,3-Bis[2-(p-dimethylaminophenyl)-2-phenylethenyl]-4,5,6,7-tetrachlorophthalide
27. 3,3-Bis[2-(p-dimethylaminophenyl)-2-(p-tert-butoxyphenyl)ethenyl]-4,5,6,7-tetrachlorophthalide
28. 3,3-Bis[2-(p-dimethylaminophenyl)-2-(p-sec-butoxyphenyl)ethenyl]-4,5,6,7-tetrachlorophthalide
29. 3,3-Bis[2-(p-methylcyclohexylaminophenyl)-2-(p-methoxyphenyl)ethenyl]-4,5,6,7-tetrachlorophthalide
30. 3,3-Bis[2-(p-dimethylaminophenyl)-2-(p-methoxyphenyl)ethenyl]-5,6-dichloro-4,7-dibromophthalide
31. 3,3-Bis[2-(p-dimethylaminophenyl)-2-(m,p-dimethylphenyl)ethenyl]-4,5,6,7-tetrachlorophthalide
32. 3,3-Bis[2-(p-dimethylaminophenyl)-2-(o-methyl-p-methoxyphenyl)ethenyl]-4,5,6,7-tetrachlorophthalide
33. 3,3-Bis[2-(p-dimethylamino-o-methylphenyl)-2-phenylethenyl]-4,5,6,7-tetrachlorophthalide
34. 3,3-Bis[2-(p-dimethylamino-o-chlorophenyl)-2-(p-methylphenyl)ethenyl]-4,5,6,7-tetrachlorophthalide
35. 3,3-Bis[2-(p-dimethylamino-m-methylphenyl)-2-(p-methoxyphenyl)ethenyl]-4,5,6,7-tetrachlorophthalide
36. 3,3-Bis[2-(p-dimethylamino-o-ethylphenyl)-2-(p-methoxyphenyl)ethenyl]-4,5,6,7-tetrachlorophthalide
37. 3,3-Bis[2-(p-dimethylaminophenyl)-2-(p-chlorophenyl)ethenyl]-4,5,6,7-tetrachlorophthalide
38. 3,3-Bis[2-(p-dimethylaminophenyl)-2-(o,p-dimethoxyphenyl)ethenyl]-4,5,6,7-tetrachlorophthalide
39. 3,3-Bis[2-(p-dimethylaminophenyl)-2-(m-methoxyphenyl)ethenyl]-4,5,6,7-tetrachlorophthalide
40. 3,3-Bis[2-(p-dipropylaminophenyl)-2-(p-methylphenyl)ethenyl]-4,5,6,7-tetrachlorophthalide
41. 3,3-Bis[2-(p-dibutylamino-o-methylphenyl)-2-(p-methoxyphenyl)ethenyl]-4,5,6,7-tetrachlorophthalide
42. 3,3-Bis[2-(p-dihexylaminophenyl)-2-phenylethenyl]-4,5,6,7-tetrachlorophthalide
43. 3,3-Bis[2-(p-dimethylaminophenyl)-2-(p-octylphenyl)ethenyl]-4,5,6,7-tetrachlorophthalide

44. 3,3-Bis[2-(p-dimethylaminophenyl)-2-(p-hexyloxyphenyl)ethenyl]-4,5,6,7-tetrachlorophthalide
45. 3,3-Bis[2-(p-methylcyclohexylaminophenyl)-2-(p-methylphenyl)ethenyl]-4,5,6,7-tetrachlorophthalide
46. 3,3-Bis[2-(p-ethylbenzylaminophenyl)-2-(p-methoxyphenyl)ethenyl]-4,5,6,7-tetrachlorophthalide
47. 3,3-Bis[2-(p-ethyltolylaminophenyl)-2-phenylethenyl]-4,5,6,7-tetrachlorophthalide
48. 3,3-Bis[2-(p-dimethylaminophenyl)-2-phenylethenyl]-4,5,6,7-tetrabromophthalide
49. 3,3-Bis[2-(p-dimethylaminophenyl)-2-(p-fluorophenyl)ethenyl]-4,5,6,7-tetrabromophthalide
50. 3,3-Bis[2-(p-dimethylaminophenyl)-2-(p-chlorophenyl)ethenyl]-4,5,6,7-tetrabromophthalide
5l. 3,3-Bis[2-(p-dimethylaminophenyl)-2-(m,p-dimethoxyphenyl)ethenyl]-4,5,6,7-tetrabromophthalide
52. 3,3-Bis[2-(p-dimethylaminophenyl)-2-(m,p-dimethylphenyl)ethenyl]-4,5,6,7-tetrabromophthalide
53. 3,3-Bis[2-(p-dimethylaminophenyl)-2-phenylethenyl]-5-chloro-4,6,7-tribromophthalide
54. 3,3-Bis[2-(p-dimethylaminophenyl)-2-(m-methylphenyl)ethenyl]-5-chloro-4,6,7-tribromophthalide
55. 3,3-Bis[2-(p-dimethylaminophenyl)-2-(p-hexyloxyphenyl)ethenyl]-5-chloro-4,6,7-tribromophthalide
56. 3,3-Bis[2-(p-dimethylaminophenyl)-2-phenylethenyl]-6-chloro-4,5,7-tribromophthalide
57. 3,3-Bis[2-(p-dimethylamino-o-chlorophenyl)-2-(p-methylphenyl)ethenyl]-6-chloro-4,5,7-tribromophthalide
58. 3,3-Bis[2-(p-dimethylaminophenyl)-2-(p-methoxyphenyl)ethenyl]-6-chloro-4,5,7-tribromophthalide
59. 3,3-Bis[2-(p-dimethylaminophenyl)-2-phenylethenyl]-5,6-dichloro-4,7-dibromophthalide
60. 3,3-Bis[2-(p-dimethylaminophenyl)-2-(m-methyl-p-methoxyphenyl)ethenyl]-5,6-dichloro-4,7-dibromophthalide
6l. 3,3-Bis[2-(p-dimethylaminophenyl)-2-(m-methoxyphenyl)ethenyl]-5,6-dichloro-4,7-dibromophthalide
62. 3,3-Bis[2-(p-dimethylaminophenyl)-2-(p-butoxyphenyl)ethenyl]-4,5,6,7-tetrabromophenylphthalide
63. 3,3-Bis[2-(p-dimethylaminophenyl)-2-(p-hexyloxyphenyl)ethenyl]-4,5,6,7-tetrabromophthalide
64. 3,3-Bis[2-(p-dimethylamino-o-methylphenyl)-2-(p-methylphenyl)ethenyl]-4,5,6,7-tetrabromophthalide
65. 3,3-Bis[2-(p-dimethylamino-m-methylphenyl)-2-(p-methoxyphenyl)ethenyl]-4,5,6,7-tetrabromophthalide
66. 3,3-Bis[2-(p-dimethylamino-m-ethoxyphenyl)-2-phenylethenyl]-4,5,6,7-tetrabromophthalide
67. 3,3-Bis[2-(p-diethylaminophenyl)-2-(p-methylphenyl)ethenyl]-4,5,6,7-tetrabromophthalide
68. 3,3-Bis[2-(p-dipropylaminophenyl)-2-(p-methoxyphenyl)ethenyl]-4,5,6,7-tetrabromophthalide
69. 3,3-Bis[2-(p-dibutylaminophenyl)-2-(p-methylphenyl)ethenyl]-4,5,6,7-tetrabromophthalide
70. 3,3-Bis[2-(p-dihexylaminophenyl)-2-(p-methylphenyl)ethenyl]-4,5,6,7-tetrabromophthalide
7l. 3,3-Bis[2-(p-methylbutylaminophenyl)-2-(p-methylphenyl)ethenyl]-4,5,6,7-tetrabromophthalide
72. 3,3-Bis[2-(p-methylcyclohexylaminophenyl)-2-(p-methoxyphenyl)ethenyl]-4,5,6,7-tetrabromophthalide
73. 3,3-Bis[2-(p-ethylbenzylaminophenyl)-2-phenylethenyl]-4,5,6,7-tetrabromophthalide
74. 3,3-Bis[2-(p-ethyltolylaminophenyl)-2-(p-methoxyphenyl)ethenyl]-4,5,6,7-tetrabromophthalide
75. 3,3-Bis[2-(p-aminophenyl)-2-(p-methoxyphenyl)ethenyl]-4,5,6,7-tetrachlorophthalide
76. 3,3-Bis[2-(p-ethylaminophenyl)-2-(p-methoxyphenyl)ethenyl]-4,5,6,7-tetrachlorophthalide
77. 3,3-Bis[2-(p-methylmethoxyethylaminophenyl)-2-(p-methoxyphenyl)ethenyl]-4,5,6,7-tetrachlorophthalide
78. 3,3-Bis[2-(p-methylethoxypropylaminophenyl)-2-(p-methoxyphenyl)ethenyl]-4,5,6,7-tetrachlorophthalide
79. 3,3-Bis[2-(p-dimethoxyethylaminophenyl)-2-(p-methylphenyl)ethenyl]-5-chloro-4,6,7-tribromophthalide
80. 3,3-Bis[2-(p-benzylmethoxypropylaminophenyl)-2-(p-ethoxyphenyl)ethenyl]-4,5,6,7-tetrachlorophthalide
8l. 3,3-Bis[2-(p-cyclohexylethoxyethylaminophenyl)-2-phenylethenyl]-5,6-dichloro-4,7-dibromophthalide
82. 3,3-Bis[2-(p-methylmethoxyethylaminophenyl)-2-(p-chlorophenyl)ethenyl]-4,5,6,7-tetrachlorophthalide
83. 3,3-Bis[2-(p-ethylmethoxyethylamino-m-methylphenyl)-2-(p-methoxyphenyl)ethenyl]-4,5,6,7-tetrachlorophthalide
84. 3,3-Bis[2-(p-methylethoxypropylamino-m-ethoxyphenyl)-2-(p-methoxyphenyl)ethenyl]-4,5,6,7-tetrachlorophthalide
85. 3,3-Bis[2-(p-methylallylaminophenyl)-2-(p-methylphenyl)ethenyl]-4,5,6,7-tetrachlorophthalide
86. 3,3-Bis[2-(p-diallylaminophenyl)-2-(p-methoxyphenyl)ethenyl]-4,5,6,7-tetrachlorophthalide
87. 3,3-Bis[2-(p-ethyl-γ-chloropropylaminophenyl)-2-(m,p-dimethoxyphenyl)ethenyl]-4,5,6,7-tetrachlorophthalide
88. 3,3-Bis[2-(p-di-γ-chloropropylaminophenyl)-2-(p-ethoxyphenyl)ethenyl]-4,5,6,7-tetrabromophthalide
89. 3,3-Bis[2-(p-cyclopentyl-γ-chloropropylaminophenyl)-2-(p-butoxyphenyl)ethenyl]-4,5,6,7-tetrachlorophthalide
90. 3,3-Bis[2-(p-methylcyclohexylmethylaminophenyl)-2-(p-methoxy-m-methylphenyl)ethenyl]-4,5,6,7-tetrachlorophthalide
9l. 3,3-Bis[2-(p-ethyl-4-methylcyclohexylaminophenyl)-2-phenylethenyl]-4,5,6,7-tetrafluorophthalide

92. 3,3-Bis[2-(p-methyl-3,3,5-trimethylcyclohexylaminophenyl)-2-(p-butylphenyl)ethenyl]-4,5,6,7-tetrachlorophthalide
93. 3,3-Bis[2-(p-4-methylbenzylmethoxypropylaminophenyl)-2-(m,p-dimethoxyphenyl)ethenyl]-4,5,6,7-tetrachlorophthalide
94. 3,3-Bis[2-(p-ethylfurfurylaminophenyl)-2-(m,p-dimethylphenyl)ethenyl]-4,5,6,7-tetrachlorophthalide
95. 3,3-Bis[2-(p-methyltetrahydrofurfurylaminophenyl)-2-(p-methoxyphenyl)ethenyl]-4,5,6,7-tetrachlorophthalide
96. 3,3-Bis[2-(p-ditetrahydrofurfurylaminophenyl)-2-(m-methyl-p-methoxyphenyl)ethenyl]-4,5,6,7-tetrachlorophthalide
97. 3,3-Bis[2-(p-benzyltetrahydrofurfurylaminophenyl)-2-(p-methoxyphenyl)ethenyl]-5-chloro-4,6,7-tribromophthalide
98. 3,3-Bis[2-(p-methyltetrahydropyrane-2-yl-methylaminophenyl)-2-(p-methoxyphenyl)ethenyl]-4,5,6,7-tetrachlorophthalide
99. 3,3-Bis[2-(p-methylmethoxyethylaminophenyl)-2-(p-methoxyphenyl)ethenyl]-4,5,6,7-tetrafluorophthalide
100. 3,3-Bis[2-(p-methylethoxypropylaminophenyl)-2-(p-methoxyphenyl)ethenyl]-4,5,6,7-tetrafluorophthalide
101. 3,3-Bis[2-(p-dimethoxyethylaminophenyl)-2-(p-methylphenyl)ethenyl]-5-chloro-4,6,7-triiodophthalide
102. 3,3-Bis[2-(p-dimethylaminophenyl)-2-(p-methoxyethoxyphenyl)ethenyl]-4,5,6,7-tetrachlorophthalide
103. 3,3-Bis[2-(p-dimethylaminophenyl)-2-(p-allyloxyphenyl)ethenyl]-4,5,6,7-tetrachlorophthalide
104. 3,3-Bis[2-(p-dimethylaminophenyl)-2-(p-chloropropyloxyphenyl)ethenyl]-4,5,6,7-tetrachlorophthalide
105. 3,3-Bis[2-(p-dimethylaminophenyl)-2-(p-cyclopentyloxyphenyl)ethenyl]-4,5,6,7-tetrachlorophthalide
106. 3,3-Bis[2-(p-dimethylaminophenyl)-2-(p-cyclohexyloxyphenyl)ethenyl]-4,5,6,7-tetrachlorophthalide
107. 3,3-Bis[2-(p-dimethylaminophenyl)-2-(p-benzyloxyphenyl)ethenyl]-4,5,6,7-tetrachlorophthalide
108. 3,3-Bis[2-(p-dimethylaminophenyl)-2-(p-phenoxyphenyl)ethenyl]-4,5,6,7-tetrachlorophthalide
109. 3,3-Bis(2-[p-dimethylaminophenyl]-2-[p-(p-methoxyphenoxy)phenyl]ethenyl}-4,5,6,7-tetrachlorophthalide
110. 3,3-Bis{2-[p-diethylaminophenyl]-2-[p-(p-methoxyphenoxy)phenyl]ethenyl}-4,5,6,7-tetrachlorophthalide

The divinyl compounds according to the present invention can be synthesized by the method shown below.

As a first step, an ethylene derivative represented by the formula (2) is synthesized from a ketone by one of the following Grignard reactions a, b and c:

a. 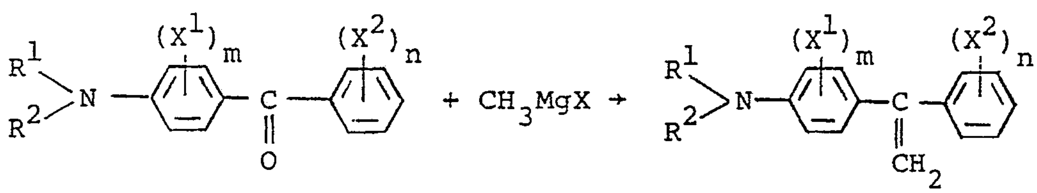 (2)

b. 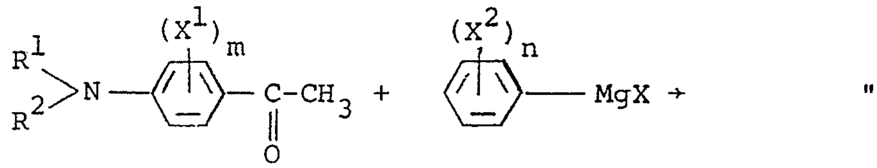 (2)

c. 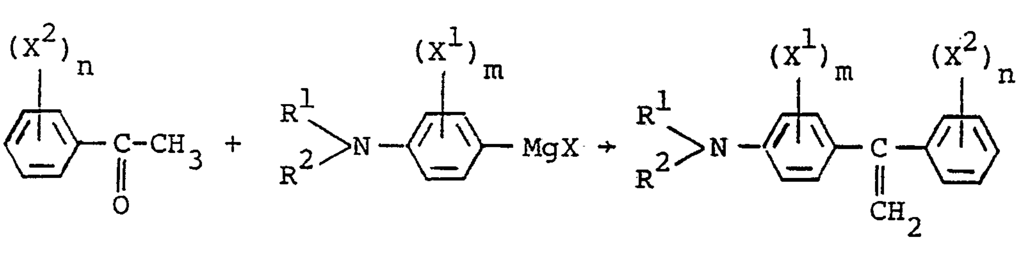 (2)

wherein $R^1$, $R^2$, $X^1$, $X^2$, m and n are as defined above and X represents a halogen atom.

The ethylene derivative of formula (2) and a phthalic acid derivative of Formula (3) (see below) are condensed in a molar ratio of 2:1 and in the presence of a dehydrating agent such as acetic anhydride or sulfuric acid, and by purifying the reaction product, the divinyl compounds represented by the formula (I) are obtained as nearly colorless crystals.

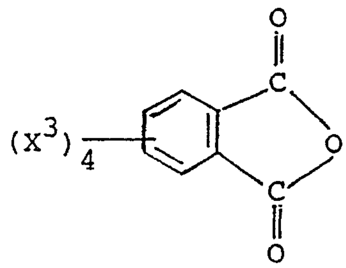 (3)

wherein $X^3$ is as defined above.

Examples of the ethylene derivative represented by the formula (2) are:

1. 1-phenyl-1-(p-dimethylaminophenyl)ethylene
2. 1-(p-methylphenyl)-1-(p-dimethylaminophenyl)ethylene
3. 1-(m,p-dimethylphenyl)-1-(p-dimethylaminophenyl)ethylene
4. 1-(p-methoxyphenyl)-1-(p-dimethylaminophenyl)ethylene
5. 1-(p-ethoxyphenyl)-1-(p-dimethylaminophenyl)ethylene
6. I-(p-methylphenyl)-I-(p-dimethylamino-o-methylphenyl)ethylene
7. I-(p-methylphenyl)-I-(p-dimethylamino-o-ethoxyphenyl)ethylene
8. I-(p-methoxyphenyl)-I-(p-dimethylamino-o-methylphenyl)ethylene
9. I-phenyl-I-(p-dimethylamino-m-methylphenyl)ethylene
I0. I-(p-methoxy-o-methylphenyl)-I-(p-dimethylaminophenyl)ethylene
II. I-(p-chlorophenyl)-I-(p-dimethylaminophenyl)ethylene
I2. I-(p-methoxyphenyl)-I-(p-dimethylamino-o-chlorophenyl)ethylene
I3. I-(p-butoxyphenyl)-I-(p-dimethylaminophenyl)ethylene
I4. I-(p-methoxyphenyl)-I-(p-diethylaminophenyl)ethylene
I5. I-(p-methylphenyl)-I-(p-dipropylamino-o-ethoxyphenyl)ethylene
I6. I-(p-ethoxyphenyl)-I-(p-dipentylaminophenyl)ethylene
I7. I-phenyl-I-(p-dioctylaminophenyl)ethylene
I8. I-(p-methylphenyl)-I-(p-methylbutylaminophenyl)ethylene
I9. I-(p-methoxyphenyl)-I-(p-methylcyclohexylaminophenyl)ethylene
20. I-(p-methylphenyl)-I-[p-ethyl-(p-methylbenzyl)aminophenyl]ethylene
2I. I-phenyl-I-[p-ethyl-(p-tolyl)aminophenyl]ethylene
22. I-(p-methoxyphenyl)-I-(p-methylaminophenyl)ethylene
23. I-(p-methoxyphenyl)-I-(p-methylmethoxyethylaminophenyl)ethylene
24. I-(p-chlorophenyl)-I-(p-ethylethoxypropylamino-m-methylphenyl)ethylene
25. I-(p-methoxyphenyl)-I-(p-diethoxyethylaminophenyl)ethylene
26. I-(m,p-dimethylphenyl)-I-(p-propyl-γ-chloropropylaminophenyl)ethylene
27. I-(p-methoxyphenyl)-I-(p-di-γ-chloropropylaminophenyl)ethylene
28. I-(m,p-dimethoxyphenyl)-I-(p-cyclohexyl-γ-chloropropylaminophenyl)ethylene
29. I-(p-ethoxyphenyl)-I-(p-ethylallylaminophenyl)ethylene
30. I-(p-isopropoxyphenyl)-I-(p-diallylaminophenyl)ethylene
3I. I-(p-methoxyphenyl)-I-(p-ethylcyclohexylmethylaminophenyl)ethylene
32. I-(p-methoxyphenyl)-I-(p-dicyclohexylmethylaminophenyl)ethylene
33. I-(p-methoxyphenyl)-I-[p-methyl-(3-methylcyclohexyl)aminophenyl]ethylene
34. I-(p-ethoxy-m-methylphenyl)-I-[p-ethyl-(3,3,5-trimethylcyclohexyl)aminophenyl]ethylene
35. I-(p-methoxyphenyl)-I-(p-methylfurfurylaminophenyl)ethylene
36. I-(p-methoxyphenyl)-I-(p-ethyltetrahydrofurfurylaminophenyl)ethylene
37. I-(m-methyl-p-ethoxyphenyl)-I-(p-ethyltetrahydropyrane-2-yl-methylaminophenyl)ethylene
38. 1-(p-methoxyphenyl)-1-(ditetrahydropyrane-2-yl-methylaminophenyl)ethylene
39. 1-phenyl-1-(p-aminophenyl)ethylene
40. 1-(m,p-dimethoxyphenyl)-1-(p-dimethylaminophenyl)ethylene

41. 1-(p-bromophenyl)-1-(p-dimethylaminophenyl)ethylene

Examples of the phthalic acid derivatives represented by the formula (3) are:

tetrachlorophthalic anhydride; 4-chloro-3,5,6-tribromophthalic anhydride; 4,5-dichloro-3,6-dibromophthalic anhydride; 4-bromo-3,5,6-trichlorophthalic anhydride; 4,5-dibromo-3,6-dichlorophthalic anhydride; tetrabromophthalic anhydride; tetrafluorophthalic anhydride; tetraiodophthalic anhydride; 4,5-dichloro-3,6-difluorophthalic anhydride; 4-chloro-3,5,6-triiodophthalic anhydride and 4,5-dichloro-3,6-diiodophthalic anhydride.

The present invention also provides a recording material incorporating, as a chromogenic agent, a divinyl compound represented by the formula (I) as defined above or which has been prepared by a process as defined above.

In case where a pressure-sensitive recording paper or a heat-sensitive recording paper, for example, is produced with these divinyl compounds, one or more of the compounds can be used. By mixing not less than two of the compounds, the chromogenic property and the image stability are improved. Moreover, to improve the hue and the concentration of developed color, and the stability of the color image, various known chromogenic agents which give various hues can be used with the present compounds to the extent that they do not damage the facilities of the present compounds.

For instance, the present compounds can be used with a chromogenic agent which has a fundamental skeleton of 3,3-bis(aminophenyl)-6-aminophthalide, 3,3-bis(indolyl)phthalide, 3-aminofluoran, aminobenzofluoran, 2,6-diaminofluoran, 2,6-diamino-3-methylfluoran, spiropyrane, phenothiazine, phenoxazine, leucoauramine, diarylcarbazolylmethane, 3-indolyl-3-(aminophenyl)azaphthalide, triaminofluorenephthalide or tetraaminodivinylphthalide.

When producing a pressure-sensitive recording paper, various solvents for the chromogenic agent can be used, such as those of the alkylbenzene series, alkylbiphenyl series, alkylnaphthalene series, diarylethane series, hydrogenated terphenyl series and chlorinated paraffin series. These can be used singly or as a mixture. For encapsulation, a coacervation method, a interfacial polymerization method or an in-situ method can be used.

As a developer, clays such as bentonite, activated clay or acid clay; metal salts of salicyclic acid, salicyclic ester derivatives or salicyclic acid derivatives,; hydroxy compounds such as 2,2-bis(p-hydroxyphenyl)propane (bisphenol A) or esters of p-hydroxybenzoic acid,; p-phenylphenol-formaldehyde resin, p-octylphenolformaldehyde resin and metal salts thereof are for example used.

As a developer, one or more of the following hydroxy compounds can, for example, be used:

p-phenylphenol, p-hydroxydiphenyl ether, methyl p-hydroxybenzoate, benzyl p-hydroxybenzoate, 2,2-bis(p-hydroxyphenyl)propane, 4,4'-thiodiphenol, bis(4-hydroxy-3-methylphenyl)sulfide, 4,4'-dihydroxydiphenylsulfone, 4-hydroxy-4'-methyldiphenylsulfone, 4-hydroxy-4'-ethyldiphenylsulfone, 3,4-dihydroxy-4'-methyldiphenylsulfone, 4-hydroxy-4'-isopropoxydiphenylsulfone, 4,4'-dihydroxy-3,3'-dimethyldiphenylsulfone, 4,4'-dihydroxy-3,3'-diallyldiphenylsulfone, 1,5-di(4-hydroxyphenylthio)-3-oxapentane, 1,7-di(4-hydroxyphenylthio)-3,5-dioxaheptane, 1,8-di(4-hydroxyphenylthio)-3,6-dioxaoctane and bis(4-hydroxy-3-methylphenyl)sulfide.

When producing a heat-sensitive recording paper, polyvinyl alcohol, methylcellulose, hydroxyethylcellulose, carboxymethylcellulose, gum arabic, gelatine, caseine, starch, polyvinyl pyrrolidone, copolymer of styrene and maleic anhydride, can, for example, be used as a binder.

Examples of sensitivity-improving agents are acetoanilide; paraffin wax; carnauba wax; higher fatty acids; esters of a higher fatty acid; amides of a higher fatty acid; phthalic esters; terephthalic esters; benzyl 4-bensyloxybenzoate; naphthol benzyl ether; 1,4-dialkoxynaphthalene; m-terphenyl; p-benzylbiphenyl; dibenzylbenzene; esters of 1-hydroxy-2-naphthoic acid; 1-phenoxy-2-naphthoxy-1-ethane; 1,2-di(3-methylphenoxy)ethane; 1-(2-isopropylphenoxy)-2-naphthoxy-2-ethane; esters of 2-hydroxy-3-naphthoic acid; 4,4'-dialkoxydiphenylsulfone; benzamide; diphenylamine; benzenesulfonamide; benzenesulfonanilide; carbazole; hydroquinone dibenzyl ether; and diphenyl carbonate. These can be used singly or after mixing together.

In order to improve the light-resistance and the preservability of the color image, it is effective to add an anti-oxidant, an anti-deteriorant or an ultraviolet absorbent, or to use a coating of a high polymeric substance.

The present invention also provides the use as a chromogenic agent of a divinyl compound represented by the formula (I) as defined above or which has been prepared by a process as defined above.

The present invention is further described in the following Examples. The Synthetic Examples illustrate the preparation of compounds represented by the formula (I). The Production Examples illustrate the preparation of chromogenic recording-materials incorporating the compound represented by the formula (I).

SYNTHETIC EXAMPLE 1:

Synthesis of 3,3-bis[2-(p-dimethylaminophenyl)-2-(p-methoxyphenyl)ethenyl]-4,5,6,7-tetrachlorophthalide

Into 25 ml of acetic anhydride, 13.0 g of 1-(p-methoxyphenyl)-1-(p-dimethylaminophenyl)ethylene and 9.3 g of tetrachlorophthalic anhydride were added and the mixture was stirred for 2 hours at 120 °C. After adding the reaction mixture to 200 ml of water and making the mixture alkaline with sodium hydroxide, the alkaline mixture was extracted with 70 ml of toluene. The solid matter obtained by distilling off the toluene from the extract was recrystallised from butanol while purifying with activated carbon to obtain 14.3 g of pale yellow crystals melting at 133-135 °C.

By elementary analysis and by the infrared absorption spectrum and the nuclear magnetic resonance spectrum of the crystals obtained, it was confirmed that the crystals were of a compound represented by the following formula:

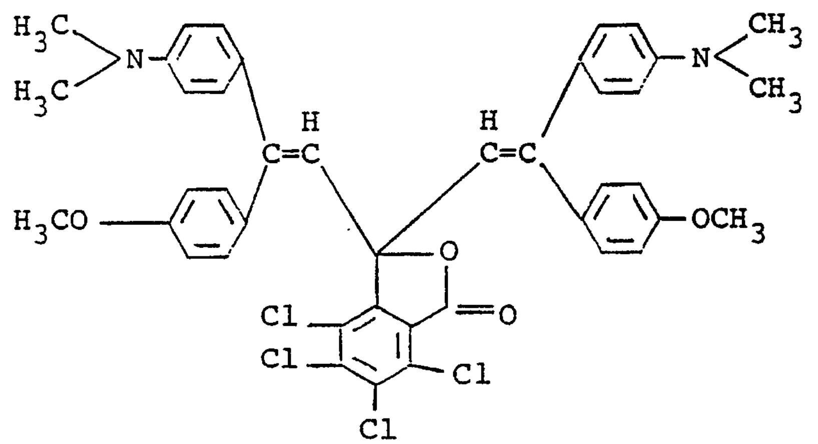

This compound was rapidly colored blue-black by activated clay. The λmax in methanol•stannic chloride was 890 nm.

The ethylene derivative, 1-(p-methoxyphenyl)-1-(p-dimethylaminophenyl)ethylene, used in the above reaction, was synthesized as follows.

Into 30 ml of ether, 4 g of metallic magnesium were added and then 0.2 ml of methyl iodide was added to the mixture. After stirring the mixture for a while, a solution prepared by dissolving 24.8 g of methyl iodide in 40 ml of ether was added to the mixture over 2 hours under a reflux condenser while stirring the mixture.

Separately, a solution was prepared by mixing 18.2 g of 4-methoxy-4'-dimethylaminobenzophenone (melting at 126-127 °C) and 100 ml of tetrahydrofurane, and the solution was slowly added to the liquid reaction mixture. The mixture was stirred for one hour at a temperature of 40 to 50 °C and then mixed with 400 ml of water and 300 ml of toluene. After making the mixture weakly acidic by dilute hydrochloric acid, the acidified mixture was stirred at 80 °C and separated into an aqueous layer and an organic layer (toluene layer). After adding activated carbon to the toluene layer and filtering the layer while hot, the toluene was distilled off from the filtrate to obtain 17.1 g of 1-(p-methoxyphenyl)-1-(p-dimethylaminophenyl)ethylene which was pale yellow in color and melted at 123-125 °C.

SYNTHETIC EXAMPLE 2:

Synthesis of 3,3-bis[2-(p-dimethylaminophenyl)-2-(p-ethoxyphenyl)ethenyl]-4,5,6,7-tetrachlorophthalide

Into a mixture of 25 ml of acetic anhydride and 75 ml of o-dichlorobenzene, 13.4 g of 1-(p-ethoxyphenyl)-1-(p-dimethylaminophenyl)ethylene (melting at 110 - 111 °C) and 21.5 g of tetrachlorophthalic anhydride were added and the mixture was stirred for 6 hours at 120 °C. After adding the reaction mixture to 200 ml of water and making the mixture alkaline by sodium hydroxide, the alkaline mixture was extracted with 70 ml of toluene. The solid matter obtained by distilling off the toluene from the extract was recrystallized from acetone while purifying with activated carbon to obtain 16.2 g of pale yellow crystals in a yield of 80.8%.

The obtained compound melted at 168 - 170 °C, and from elementary analysis, the infrared absorption spectrum and the nuclear magnetic resonance spectrum, it was confirmed that the compound was one

represented by the following formula:

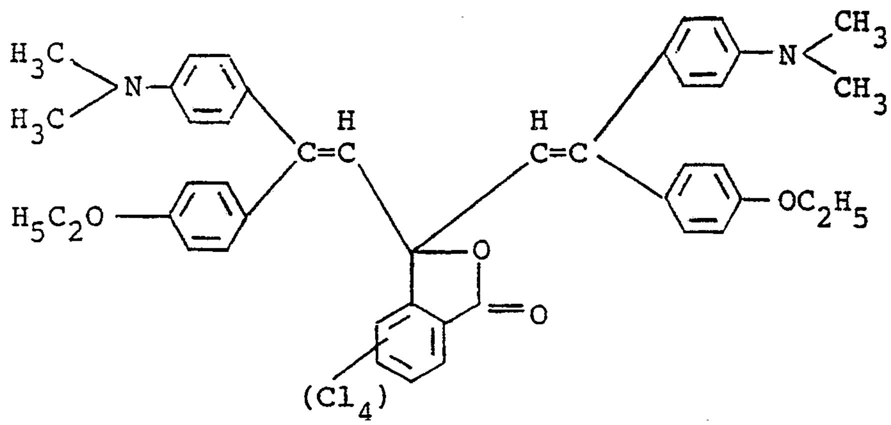

This compound was rapidly colored blue-black by activated clay. The λmax of this compound in methanol•stannic chloride was 882 nm.

SYNTHETIC EXAMPLES 3 - 74:

By reacting various ethylene derivatives with various phthalic acid derivatives in the same manner as in Synthetic Examples 1 and 2, the divinyl compounds shown in Table 1 were synthesized. All the compounds were solid and colorless to pale yellow in color. They were colored rapidly into the hue shown in Table 1.

For preparing a pressure-sensitive recording paper with the divinyl compounds represented by the formula (I), any known method can be used, for instance, the coacervation method disclosed in US-A-2,800,458 and US-A-2,806,457. For preparing the heat-sensitive recording paper, a known method, for instance the method disclosed in JP-B-45-14039/1960, can be used.

Table 1 (I)

| Compound No. | $R^1$ | $R^2$ | $X^1$ | $X^2$ | $X^3$ | Color | λmax (nm) | M.P. (°C) |
|---|---|---|---|---|---|---|---|---|
| 3 | $C_2H_5$- | $C_2H_5$- | - (m=0) | p-$CH_3O$- | Cl | Blue black | 900 | 95 (decomposition) |
| 4 | $CH_3$- | $CH_3$- | - (m=0) | p-n-$C_3H_7O$- | Cl | Blue black | 888 | 162 - 164 |
| 5 | $CH_3$- | $CH_3$- | - (m=0) | p-i-$C_3H_7O$- | Cl | Blue black | 883 | 129 - 132 |
| 6 | $CH_3$- | $CH_3$- | - (m=0) | p-n-$C_4H_9O$- | Cl | Blue black | 885 | 180 - 183 |
| 7 | $CH_3$- | $CH_3$- | - (m=0) | p-i-$C_4H_9O$- | Cl | Blue black | 885 | 179 - 182 |
| 8 | $CH_3$- | $C_2H_5$- | - (m=0) | p-$CH_3O$- | Cl | Blue black | 892 | 120 (decomposition) |
| 9 | $CH_3$- | $C_3H_7$- | - (m=0) | p-$CH_3O$- | Cl | Blue black | 895 | Difficult to crystallize |
| 10 | $CH_3$- | $CH_3$- | - (m=0) | p-$CH_3$- | Cl | Black | 900 | 215 - 218 |
| 11 | $C_2H_5$- | $C_3H_7$- | - (m=0) | p-$CH_3O$- | Cl | Blue Black | 895 | 115 - 118 |

Table 1 (II)

| Compound No. | $R^1$ | $R^2$ | $X^1$ | $X^2$ | $X^3$ | Color | λmax (nm) | M.P. (°C) |
|---|---|---|---|---|---|---|---|---|
| 12 | $CH_3$- | i-$C_3H_7$- | - (m=0) | p-$CH_3$O- | Cl | Blue black | 895 | Difficult to crystallize |
| 13 | $C_2H_5$- | i-$C_3H_7$- | - (m=0) | p-$CH_3$O- | Cl | Blue black | 895 | 136 - 139 |
| 14 | $C_3H_7$- | $C_3H_7$- | - (m=0) | p-$CH_3$O- | Cl | Blue black | 897 | Difficult to crystallize |
| 15 | $CH_3$- | $C_4H_9$- | - (m=0) | p-$CH_3$O- | Cl | Blue black | 895 | 105 - 108 |
| 16 | $C_2H_5$- | $C_4H_9$- | - (m=0) | p-$CH_3$O- | Cl | Blue black | 892 | Difficult to crystallize |
| 17 | $C_2H_5$- | $C_2H_5$- | - (m=0) | p-$C_2H_5$O- | Cl | Blue black | 895 | 125 - 128 |
| 18 | $CH_3$- | $CH_3$- | - (m=0) | p-$CH_3$O-, m-$CH_3$- | Cl | Blue black | 882 | 131 - 135 |
| 19 | $CH_3$- | $CH_3$- | - (m=0) | p-n-$C_5H_{11}$O- | Cl | Blue black | 882 | Difficult to crystallize |
| 20 | $CH_3$- | $CH_3$- | - (m=0) | p-$CH_3$O- | Br | Blue black | 900 | 133 - 135 |

Table 1 (III)

| Compound No. | $R^1$ | $R^2$ | $X^1$ | $X^2$ | $X^3$ | Color | λmax (nm) | M.P. (°C) |
|---|---|---|---|---|---|---|---|---|
| 21 | $CH_3-$ | $CH_3-$ | - (m=0) | p-i-$C_5H_{11}O-$ | Cl | Blue black | 880 | 125 - 128 |
| 22 | $CH_3-$ | $CH_3-$ | - (m=0) | p-$CH_3O-$, m-$CH_3O-$ | Cl | Blue black | 890 | 208 - 211 |
| 23 | $CH_3-$ | $C_2H_5-$ | - (m=0) | p-$C_2H_5O-$ | Cl | Blue black | 890 | 138 - 141 |
| 24 | $CH_3-$ | $C_3H_7-$ | - (m=0) | p-$C_2H_5O-$ | Cl | Blue black | 890 | 118 - 121 |
| 25 | $CH_3-$ | $CH_3-$ | - (m=0) | p-$C_2H_5-$ | Cl | Black | 900 | 185 - 188 |
| 26 | $CH_3-$ | $CH_3-$ | - (m=0) | - (n=0) | Cl | Black | 905 | 120 - 125 |
| 27 | $CH_3-$ | $CH_3-$ | - (m=0) | p-t-$C_4H_9O-$ | Cl | Blue black | 885 | Difficult to crystallize |
| 28 | $CH_3-$ | $CH_3-$ | - (m=0) | p-s-$C_4H_9O-$ | Cl | Blue black | 885 | 165 - 168 |
| 29 | $CH_3-$ | 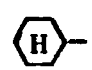 | - (m=0) | p-$CH_3O-$ | Cl | Blue black | 890 | 165 (decomposition) |

Table 1 (IV)

| Compound No. | $R^1$ | $R^2$ | $X^1$ | $X^2$ | $X^3$ | Color | λmax (nm) | M.P. (°C) |
|---|---|---|---|---|---|---|---|---|
| 30 | $CH_3-$ | $CH_3-$ | - (m=0) | $p-CH_3O-$ | $5,6-Cl_2-$, $4,7-Br_2-$ | Blue black | 884 | 145 - 148 |
| 31 | $CH_3-$ | $CH_3-$ | - (m=0) | p-Cl- | Cl | Black | 915 | 133 - 135 |
| 32 | $CH_3-$ | $CH_3-$ | - (m=0) | $m-CH_3-$, $p-CH_3-$ | Cl | Black | 900 | 150 - 155 |
| 33 | $C_2H_5-$ | $C_2H_5-$ | $O-CH_3-$ | $p-CH_3-$ | Cl | Black | 900 | Difficult to crystallize |
| 34 | $C_2H_5-$ | $C_2H_5-$ | $O-C_3H_7O-$ | $p-CH_3O-$ | Cl | Violet-ish black | 820 | Difficult to crystallize |
| 35 | $C_4H_9-$ | $C_4H_9-$ | - (m=0) | $p-CH_3-$ | Cl | Black | 910 | 135 - 138 |
| 36 | $C_2H_5-$ | $C_5H_{11}-$ | - (m=0) | $p-CH_3O-$ | Cl | Blue black | 890 | Difficult to crystallize |
| 37 | $C_6H_{13}-$ | $C_6H_{13}-$ | - (m=0) | $p-CH_3-$ | Cl | Black | 910 | Difficult to crystallize |
| 38 | $C_2H_5-$ | 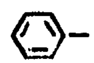 $-CH_2-$ | - (m=0) | - (n=0) | Cl | Black | 920 | Difficult to crystallize |

Table 1 (V)

| Compound No. | $R^1$ | $R^2$ | $X^1$ | $X^2$ | $X^3$ | Color | λmax (nm) | M.P. (°C) |
|---|---|---|---|---|---|---|---|---|
| 39 | $C_2H_5$- | $CH_3$ 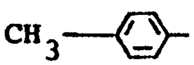 | - (m=0) | p-$CH_3$- | Cl | Black | 910 | Difficult to crystallize |
| 40 | $CH_3$- | $CH_3$- | - (m=0) | p-$CH_3O$- | 5-Cl-, $(Br)_3$- | Blue black | 885 | 130 - 135 |
| 41 | $CH_3$- | $CH_3$- | - (m=0) | p-$C_4H_9O$- | 5,6-$(Cl)_2$-, 4,7-$(Br)_2$- | Blue black | 882 | 178 - 181 |
| 42 | $CH_3$- | $CH_3$- | - (m=0) | p-$C_6H_{13}O$- | Br | Blue black | 882 | Difficult to crystallize |
| 43 | $CH_3$- | $CH_3$- | - (m=0) | - (n=0) | Br | Black | 910 | 145 - 148 |
| 44 | $CH_3$- | $CH_3$- | - (m=0) | p-$CH_3$- | Br | Black | 890 | 224 - 226 |
| 45 | $CH_3$- | $CH_3$- | - (m=0) | p-$CH_3$- | 5-Cl-, $(Br)_3$- | Black | 900 | 218 - 222 |
| 46 | $CH_3$- | $CH_3$- | - (m=0) | p-t-$C_4H_9$- | Cl | Black | 895 | 198 - 200 |
| 47 | $CH_3$- | $CH_3$- | - (m=0) | m-$CH_3O$-, p-$CH_3O$- | Br | Blue Black | 890 | 136 - 140 |

Table 1 (VI)

| Compound No. | $R^1$ | $R^2$ | $X^1$ | $X^2$ | $X^3$ | Color | λmax (nm) | M.P. (°C) |
|---|---|---|---|---|---|---|---|---|
| 48 | $C_2H_5$- | $C_2H_5$- | - (m=0) | p-$CH_3O$- | Br | Blue black | 903 | Difficult to crystallize |
| 49 | $C_2H_5$- | $C_2H_5$- | - (m=0) | p-$CH_3$- | Br | Black | 905 | 131 - 135 |
| 50 | $C_2H_5$- | $C_2H_5$- | m-$CH_3$- | p-$CH_3O$- | Br | Blue black | 910 | Difficult to crystallize |
| 51 | $C_4H_9$- | $C_4H_9$- | - (m=0) | p-$C_2H_5O$- | Br | Blue black | 900 | Difficult to crystallize |
| 52 | $C_2H_5$- | i-$C_5H_{11}$- | - (m=0) | p-t-$C_4H_9$- | Br | Black | 905 | Difficult to crystallize |
| 53 | $C_2H_5$- | 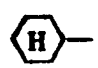 | - (m=0) | p-$CH_3O$- | Br | Blue black | 900 | 165 - 168 |
| 54 | $CH_3$- | 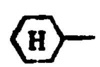 | - (m=0) | p-$CH_3O$- | Br | Blue black | 910 | 185 - 188 |
| 55 | $C_2H_5$- | $CH_3$- 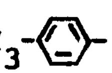 $CH_2$- | m-$CH_3O$- | m-$(CH_3)$-, p-$(CH_3)$- | 5-Cl-, $(Br)_3$- | Black | 900 | Difficult to crystallize |
| 56 | $CH_3$- | $CH_3OC_2H_4$- | - (m=0) | p-$CH_3O$- | Cl | Blue black | 885 | 95 (decomposition) |

Table 1 (VII)

| Compound No. | $R^1$ | $R^2$ | $X^1$ | $X^2$ | $X^3$ | Color | λmax (nm) | M.P. (°C) |
|---|---|---|---|---|---|---|---|---|
| 57 | $CH_3$- | $CH_2$=CH-$CH_2$- | - (m=0) | p-$CH_3$- | Cl | Reddish black | 900 | 113 - 115 |
| 58 | $C_2H_5$- | $CH_3OC_3H_6$- | - (m=0) | p-$CH_3O$- | Br | Blue black | 880 | Difficult to crystallize |
| 59 | $CH_3$- | $C_2H_5OC_3H_6$- | - | p-$CH_3O$-, m-$CH_3O$- | Cl | Blue black | 887 | 112 - 115 |
| 60 | $CH_3OC_2H_4$- | $CH_3OC_2H_4$- | - (m=0) | p-Cl- | Cl | Reddish black | 920 | Difficult to crystallize |
| 61 | $CH_3$- | $CH_3OC_3H_6$- | O-$CH_3$- | p-$CH_3O$- | Cl | Blue black | 880 | Difficult to crystallize |
| 62 | $CH_3$- | 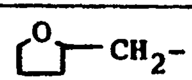 | - (m=0) | p-$C_2H_5O$-, m-$CH_3$- | Cl | Blue black | 890 | 125 - 129 |
| 63 | $C_2H_5$- | 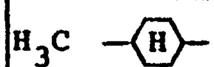 | - (m=0) | p-t-$C_4H_9$- | Cl | Black | 897 | Difficult to crystallize |
| 64 | $CH_3$- | $CH_2$=CH-$CH_2$- | - (m=0) | p-$CH_3$- | Br | Reddish black | 892 | 116 - 120 |
| 65 | $CH_3$ | $CH_2$=CH-$CH_2$- | - (m=0) | p-$CH_3$- | 5-Cl $(Br)_3$- | Reddish black | 893 | 108 - 112 |

Table 1 (VIII)

| Compound No. | $R^1$ | $R^2$ | $X^1$ | $X^2$ | $X^3$ | Color | λmax (nm) | M.P. (°C) |
|---|---|---|---|---|---|---|---|---|
| 66 | $CH_3-$ | $CH_2=CH-CH_2-$ | - (m=0) | $p-CH_3-$ | 5,6-$Cl_2$-4,7-$Br_2$- | Reddish black | 895 | 105 - 108 |
| 67 | $CH_3-$ | $CH_2=CH-CH_2-$ | - (m=0) | $p-CH_3-$ | F | Reddish black | 891 | Difficult to crystallize |
| 68 | H- | $CH_3-$ | - (m=0) | $p-CH_3O-$ | Cl | Blue black | 870 | Difficult to crystallize |
| 69 | $CH_3-$ | $CH_3-$ | - (m=0) | p-($CH_3O$- 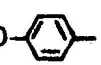 -O)- | Cl | Blue black | 892 | 83 (decomposition) |
| 70 | $CH_3-$ | $CH_3-$ | - (m=0) | $p-CH_3OC_2H_4O-$ | Cl | Blue black | 890 | 145 - 148 |
| 71 | $CH_3-$ | $CH_3-$ | - (m=0) | p-( 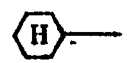 - O)- | Cl | Blue black | 895 | 151 - 154 |
| 72 | $CH_3-$ | $CH_3-$ | - (m=0) | p-( 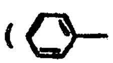 - $CH_2$-O)- | Cl | Blue black | 897 | 168 - 171 |
| 73 | $CH_3-$ | $CH_3-$ | - (m=0) | p-($CH_3O$- 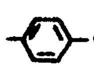 -O)- | Br | Blue black | 895 | Difficult to crystallize |
| 74 | $CH_3-$ | $CH_3-$ | - (m=0) | p-($C_8H_{17}O$)- | Cl | Blue black | 893 | Difficult to crystallize |

PRODUCTION EXAMPLE 1:

Production of a pressure-sensitive copying paper.

Into 95 parts by weight of monoisopropylbiphenyl, 5 parts by weight of the compound of Example 2, namely, 3,3-bis[2-(p-dimethylaminophenyl)-2-(p-ethoxyphenyl)ethenyl]-4,5,6,7-tetrachlorophthalide, were dissolved. A solution of 24 parts by weight of gelatine and 24 parts by weight of gum arabic in 400 parts by weight of water, of which the pH was adjusted to 7, was added to the monoisopropylbiphenyl solution and the mixture was emulsified by a homogenizer. Into the emulsion, 100 parts by weight of warm water were added and after stirring the mixture for 30 minutes at 50 °C about one part by weight of an aqueous 10% solution of sodium hydroxide was added. The mixture was further stirred for another 30 minutes at the same temperature.

In the next step, dilute acetic acid was added to the mixture to adjust the pH to 4.5 and after stirring for about one hour at 50 °C the mixture was cooled to 0 to 5 °C and stirred for 30 minutes. Then, 35 parts by weight of an aqueous 4% solution of glutaraldehyde were slowly added to the mixture to harden the resulting capsules and pH of the mixture was adjusted to 6 by adding a dilute aqueous solution of sodium hydroxide to complete the capsulation. During the operations, no coloring was observed.

The capsule suspension obtained was uniformly coated on a sheet of paper by a wire-bar in an amount such that the coated weight of capsules after drying was 6 $g/m^2$. The sheet was dried to obtain a capsule-coated paper sheet (the upper paper sheet).

On placing the upper paper sheet on a sheet of paper coated with a phenol-formaldehyde resin as a developer and applying writing pressure by a ball-pen on the sheets of paper, deep black letters rapidly appeared on the sheets.

The color image had excellent light-resistance and moisture-resistance and since the image had a strong absorption in the range of 800 to 1000 nm, it was possible to read the letters by OCR. Furthermore, the surface of the paper coated with the capsules had excellent light-resistance, and its color and chromogenic ability were not reduced by sunlight.

COMPARATIVE EXAMPLE 1:

In the same manner as in Production Example 1 except for using 5 parts by weight of the compound (D) as the chromogenic agent, a pressure-sensitive copying paper was prepared.

On subjecting the pressure-sensitive copying paper to color-development by a lower paper sheet on which a phenolformaldehyde resin had been applied, a light green image appeared slowly.

As the absorption of near infrared rays by the image was weak, it was difficult to read it by OCR (refer to Figure 1).

PRODUCTION EXAMPLE 2:

Production of a heat-sensitive recording paper:

1) Preparation of a liquid dispersion of a chromogenic agent (A-liquid).

A mixture of the following composition was pulverized by a paintshaker (made by TOYO-SEIKI Co., Ltd.) until the mean diameter of the particles of the chromogenic agent became 2 µm:

5 parts by weight of 3,3-bis[2-(1-p-dimethylaminophenyl)-2-(p-methoxyphenyl)ethenyl]-4,5,6,7-tetrachlorophthalide (Synthetic Example 1)
15 parts by weight of kaolin,
100 parts by weight of an aqueous 10% solution of polyvinyl alcohol and
85 parts by weight of water

2) Preparation of a liquid dispersion of a developer and a sensitizer (B-liquid).

A mixture of the following composition was pulverised by a paintshaker until the mean diameter of the particles of developer and sensitizer became 3 µm:

15 parts by weight of bisphenol A,
10 parts by weight of zinc stearate,
15 parts by weight of stearic amide and
150 parts by weight of an aqueous 10% solution of polyvinyl alcohol.

3) Preparation and application of a liquid heat-sensitive material.

By mixing together 10 parts by weight of the A-liquid and 6.5 parts by weight of the B-liquid, a liquid heat-sensitive material was obtained. The liquid material was coated on a sheet of paper by a wire-bar

uniformly in an amount such that the coated weight of solid materials after drying became 6 $g/m^2$. The sheet of paper was dried to obtain a heat-sensitive recording paper.

The heat-sensitive recording paper was nearly colorless and did not show any spontaneous coloring (refer to Figure 3). The heat-sensitive recording paper showed a dark-black color after heating with a heated pen. The color image obtained had excellent light-resistance and moisture-resistance and as the color image had a strong absorption in the range of 700 to 1050 nm, it was possible to read the image by OCR.

The same results have been obtained using the compounds in other Synthetic Examples.

COMPARATIVE EXAMPLE 2:

In the same manner as in Production Example 2 except for using 5 parts by weight of the compound (A), a heat-sensitive recording paper was obtained. Although the heat-sensitive recording paper was colored black by heating with a heated pen, as the color image did not absorb any near infrared rays, it was impossible to read the color image by OCR (refer to Figure 2).

COMPARATIVE EXAMPLE 3:

In the same manner as in Production Example 2 except for using 5 parts by weight of the compound (B), a heat-sensitive recording paper was obtained. The heat-sensitive recording paper showed yellowish green spontaneous coloring. On heating the paper with a heated pen, a green color developed (refer to Figures 2 and 3).

From the above Production Examples and Comparative Examples, it is confirmed that the divinyl compound according to the present invention is an excellent chromogenic agent for recording materials.

## Claims

**1.** A divinyl compound represented by the formula (I):

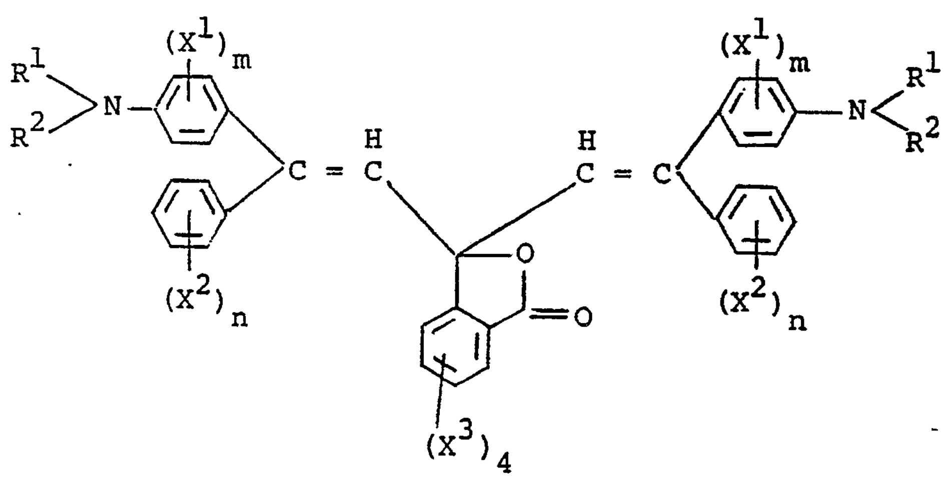

(I)

wherein $X^1$ represents an alkyl group, an alkoxy group or a halogen atom; $X^2$ represents an alkyl group, an alkoxy group, a halogen atom or $R^3O$-; $X^3$ represents a halogen atom; $R^1$ represents a hydrogen atom, an alkyl group, an alkoxyalkyl group, a haloalkyl group, an alkenyl group, an aralkyl group, a cyclohexylmethyl group, a methylcyclohexyl group, a trimethylcyclohexyl group, a furfuryl group, a tetrahydrofurfuryl group or a tetrahydropyran-2-yl-methyl group; $R^2$ represents any group defined by $R^1$ above, a cycloalkyl group, a phenyl group, a benzyl group or a phenyl or a benzyl group substituted by chlorine atom(s), bromine atom(s) or alkyl group(s) which have not more than four carbon atoms or by an alkoxy group which has not more than four carbon atoms; $R^3$ represents an aryl group, a cycloalkyl group, an alkoxyalkyl group, an alkenyl group, a haloalkyl group or an aralkyl group; and m and n each represents 0, 1, 2 or 3; wherein each $X^1$ or each $X^2$ is the same or different when m or n respectively is 2 or 3 and each $X^3$ is the same or different.

**2.** A divinyl compound according to claim 1, wherein $R^1$ represents an alkyl group having not more than six carbon atoms, an alkoxyalkyl group having not more than six carbon atoms, a haloalkyl group having not more than six carbon atoms, an alkenyl group having not more than six carbon atoms, a cyclohexyl group, a cyclohexylmethyl group, a methylcyclohexyl group, a trimethylcyclohexyl group, a furfuryl group, a tetrahydrofurfuryl group, a tetrahydropyrane-2-yl-methyl group, a benzyl group or a benzyl group substituted with an alkyl group having not more than four carbon atoms; $R^2$ represents any group defined by $R^1$ above, a phenyl group, a phenyl group substituted with a chlorine atom or an alkyl group having not more than four carbon atoms; $X^1$ represents a methyl group, a methoxy group or an ethoxy group; $X^2$ represents a halogen atom, an alkyl group having not more than six carbon atoms, an alkoxy group having not more than six carbon atoms, an alkoxyalkoxy group having not more than six carbon atoms, a cyclohexyloxy group, a benzyloxy group, a phenoxy group or a phenoxy or a benzyloxy group substituted with an alkyl or an alkoxy group both of which have not more than four carbon atoms; m is 0 or 1 and n is 0, 1 or 2.

**3.** A divinyl compound according to claim 2, wherein $R^1$ represents an alkyl group having not more than six carbon atoms; $R^2$ represents an alkyl group having not more than six carbon atoms or a cyclohexyl group; $X^2$ represents a halogen atom, an alkyl group having not more than six carbon atoms, an alkoxy group having not more than six carbon atoms, an alkoxyalkoxy group having not more than six carbon atoms, a cyclohexyloxy group, a phenoxy group, a benzyloxy group or a phenoxy or benzyloxy group substituted with an alkyl or an alkoxy group both of which have not more than four carbon atoms; $X^3$ represents a chlorine atom, m is 0 and n is 0, 1 or 2.

**4.** A divinyl compound according to claim 3, wherein $R^1$ represents a methyl group or an ethyl group; $R^2$ represents a methyl group, an ethyl group, an n-propyl group or an iso-propyl group; $X^2$ represents a methyl group, an alkoxy group having not more than five carbon atoms and n is 1 or 2.

**5.** 3,3-bis[2-(p-dimethylaminophenyl)-2-(p-methoxyphenyl)ethenyl]-4,5,6,7-tetrachlorophthalide.

**6.** 3,3-bis[2-(p-dimethylaminophenyl)-2-(p-ethoxyphenyl)ethenyl]-4,5,6,7-tetrachlorophthalide.

**7.** 3,3-bis[2-(p-diethylaminophenyl)-2-(methoxyphenyl)ethenyl]-4,5,6,7-tetrachlorophthalide.

**8.** 3,3-bis[2-(p-dimethylaminophenyl)-2-(p-propoxyphenyl)ethenyl]-4,5,6,7-tetrachlorophthalide.

**9.** 3,3-bis[2-(p-dimethylaminophenyl)-2-(p-iso-propoxyphenyl)ethenyl]-4,5,6,7-tetrachlorophthalide.

**10.** 3,3-bis[2-(p-dimethylaminophenyl)-2-(p-butoxyphenyl)ethenyl]-4,5,6,7-tetrachlorophthalide.

**11.** 3,3-bis[2-(p-dimethylaminophenyl)-2-(p-iso-butoxyphenyl)ethenyl]-4,5,6,7-tetrachlorophthalide.

**12.** 3,3-bis[2-(p-methylethylaminophenyl)-2-(p-methoxyphenyl)ethenyl]-4,5,6,7-tetrachlorophthalide.

**13.** 3,3-bis[2-(p-methylpropylaminophenyl)-2-(p-methoxyphenyl)ethenyl]-4,5,6,7-tetrachlorophthalide.

**14.** 3,3-bis[2-(p-dimethylaminophenyl)-2-(p-methylphenyl)ethenyl]-4,5,6,7-tetrachlorophthalide.

**15.** 3,3-bis[2-(p-ethylpropylaminophenyl)-2-(p-methoxyphenyl)ethenyl]-4,5,6,7-tetrachlorophthalide.

**16.** 3,3-bis[2-(p-methyl-isopropylaminophenyl)-2-(p-methoxyphenyl)ethenyl]-4,5,6,7-tetrachlorophthalide.

**17.** 3,3-bis[2-(p-ethyl-isopropylaminophenyl)-2- (p-methoxyphenyl)ethenyl] -4,5,6,7-tetrachlorophthalide.

**18.** 3,3-bis[2-(p-dipropylaminophenyl)-2-(p-methoxyphenyl)ethenyl]-4,5,6,7-tetrachlorophthalide.

**19.** 3,3-bis[2-(p-methylbutylaminophenyl)-2-(p-methoxyphenyl)ethenyl]-4,5,6,7-tetrachlorophthalide.

**20.** 3,3-bis[2-(p-ethylbutylaminophenyl)-2-(p-methoxyphenyl)ethenyl]-4,5,6,7-tetrachlorophthalide.

21. 3,3-bis[2-(p-diethylaminophenyl)-2-(p-ethoxyphenyl)ethenyl]-4,5,6,7-tetrachlorophthalide.

22. 3,3-bis[2-(p-dimethylaminophenyl)-2-(m-methyl-p-methoxyphenyl)ethenyl]-4,5,6,7-tetrachlorophthalide.

23. 3,3-bis[2-(p-dimethylaminophenyl)-2-(p-pentyloxyphenyl)ethenyl]-4,5,6,7-tetrachorophthalide.

24. 3,3-bis[2-(p-dimethylaminophenyl)-2-(p-methoxyphenyl)ethenyl]-4,5,6,7-tetrabromophthalide.

25. 3,3-bis[2-(p-dimethylaminophenyl)-2-(p-iso-pentyloxyphenyl)ethenyl]-4,5,6,7-tetrachlorophthalide.

26. 3,3-bis[2-(p-dimethylaminophenyl)-2-(m,p-dimethoxyphenyl)ethenyl]-4,5,6,7-tetrachlorophthalide.

27. 3,3-bis[2-(p-methylethylaminophenyl)-2-(p-ethoxyphenyl)ethenyl]-4,5,6,7-tetrachlorophthalide.

28. 3,3-bis[2-(p-methylpropylaminophenyl)-2-(p-ethoxyphenyl)ethenyl]-4,5,6,7-tetrachlorophthalide.

29. 3,3-bis[2-(p-dimethylaminophenyl)-2-(p-ethylphenyl)ethenyl]-4,5,6,7-tetrachlorophthalide.

30. 3,3-bis[2-(p-dimethylaminophenyl)-2-phenylethenyl]-4,5,6,7-tetrachlorophthalide.

31. 3,3-bis[2-(p-dimethylaminophenyl)-2-(p-tert-butoxyphenyl)ethenyl]-4,5,6,7-tetrachlorophthalide.

32. 3,3-bis[2-(p-dimethylaminophenyl)-2-(p-sec-butoxyphenyl)ethenyl]-4,5,6,7-tetrachlorophthalide.

33. 3,3-bis[2-(p-methylcyclohexylaminophenyl)-2-(p-methoxyphenyl)ethenyl]-4,5,6,7-tetrachlorophthalide.

34. 3,3-bis[2-(p-dimethylaminophenyl)-2-(p-methoxyphenyl)ethenyl]-5,6-dichloro-4,7-dibromophthalide.

35. 3,3-bis[2-(p-methylmethoxyethylaminophenyl)-2-(p-methoxyphenyl)ethenyl]-4,5,6,7-tetrachlorophthalide.

36. 3,3-bis[2-(p-methylallylaminophenyl)-2-(p-methylphenyl)ethenyl]-4,5,6,7-tetrachlorophthalide.

37. 3,3-bis[2-(p-dimethylamino-phenyl)-2-[p-(p-methoxyphenoxy)phenyl] ethenyl]-4,5,6,7-tetrachlorophthalide.

38. A process for the preparation of a divinyl compound represented by the formula (I) as defined in any one of the preceding claims, which process comprises condensing an ethylene derivative of formula (2):

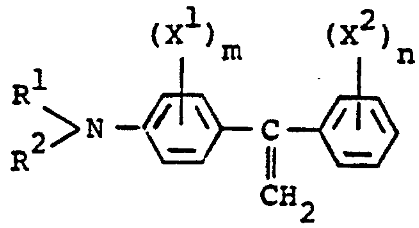

(2)

wherein $R^1$, $R^2$, $X^1$, $X^2$, m and n are as defined in claim 1, with a phthalic acid derivative of formula (3)

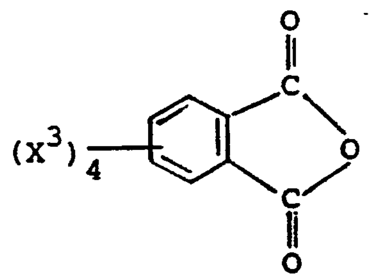

(3)

wherein $X^3$ is as defined in claim 1, in a molar ratio of 2:1 and in the presence of a dehydrating agent.

**39.** A process according to claim 38, wherein the ethylene derivative of formula (2) has been prepared by means of a Grignard reaction between the compounds $CH_3MgX$ and

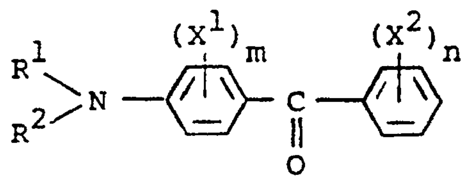

wherein X represents a halogen atom and $R^1$, $R^2$, $X^1$, $X^2$, m and n are as defined in claim 38.

**40.** A process according to claim 38, wherein the ethylene derivative of formula (2) has been prepared by means of a Grignard reaction between the compounds

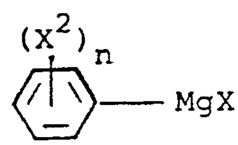

and

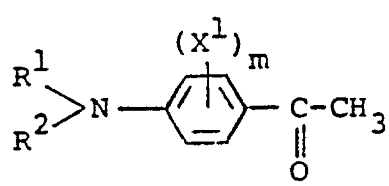

wherein X represents a halogen atom and $R^1$, $R^2$, $X^1$, $X^2$, m and n are as defined in claim 38.

**41.** A process according to claim 38, wherein the ethylene derivative of formula (2) has been prepared by means of a Grignard reaction between the compounds

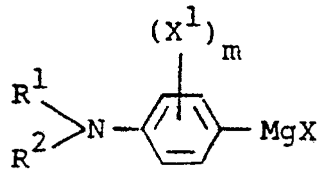

and

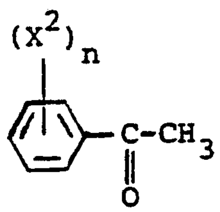

wherein X represents a halogen atom and $R^1$, $R^2$, $X^1$, $X^2$, m and n are as defined in claim 38.

**42.** A recording material incorporating, as a chromogenic agent, a divinyl compound represented by the formula (I) as claimed in any one of claims 1 to 37 or which has been prepared by a process as claimed in any one of claims 38 to 41.

**43.** A recording material according to claim 42, which is a pressure-sensitive recording paper wherein the divinyl compound is encapsulated in solution within microcapsules present on a face of a paper sheet and an opposing face of another paper sheet is coated with a developer.

**44.** A recording material according to claim 42, which is a heat-sensitive recording paper wherein a mixture comprising the divinyl compound, a binder and a developer coats a face of a paper sheet.

**45.** Use as a chromogenic agent of a divinyl compound represented by the formula (I) as claimed in any one of claims 1 to 37 or which has been prepared by a process as claimed in any one of claims 38 to 41.

**46.** A recording material according to any one of claims 42 to 44 incorporating at least two said divinyl compounds.

**47.** A recording material according to any one of claims 42 to 44 or 46, further comprising an additional chromogenic agent having the fundamental skeleton of 3,3-bis-(aminophenyl)-6-aminophthalide, 3,3-bis-(indolyl)-phthalide, 3-aminofluoran, aminobenzofluoran, 2,6-diaminofluoran, 2,6-diamino-3-methylfluoran, spiropyrane, phenothiazine, phenoxazine, leucoauramine, diarylcarbazolylmethane, 3-indolyl-3-(aminophenyl)-azaphthalide, triamino-fluorenephthalide or tetraaminodivinylphthalide.

**Revendications**

**1.** Composé divinylique de formule (I):

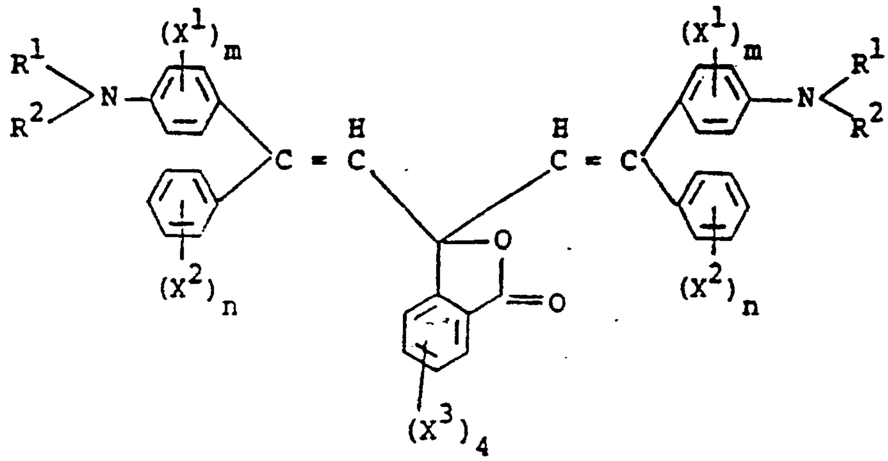

(I)

dans laquelle $X^1$ représente un groupe alkyle, un groupe alkoxy ou un atome d'halogène; $X^2$ représente un groupe alkyle, un groupe alkoxy, un atome d'halogène ou un groupe $R^3O$-; $X^3$ représente un atome d'halogène; $R^1$ représente un atome d'hydrogène, un groupe alkyle, un groupe alkoxyalkyle, un groupe haloalkyle, un groupe alkényle, un groupe aralkyle, un groupe cyclohexylméthyle, un groupe méthylcyclohexyle, un groupe triméthylcyclohexyle, un groupe furfuryle, un groupe tétrahydrofurfuryle ou un groupe tétrahydropyran-2-yl-méthyle; $R^2$ représente n'importe quel groupe $R^1$ défini ci-dessus, un groupe cycloalkyle, un groupe phényle, un groupe benzyle, ou un groupe phényle ou benzyle substitué avec un ou plusieurs atomes de chlore, un ou plusieurs atomes de brome, un ou plusieurs groupes alkyle ne comportant pas plus de 4 atomes de carbone ou un groupe alkoxy ne comportant pas plus de 4 atomes de carbone; $R^3$ représente un groupe aryle, un groupe cycloalkyle, un groupe alkoxyalkyle, un groupe alkényle, un groupe haloalkyle ou un groupe aralkyle; et m et n représentent chacun 0,1,2 ou 3; les groupes $X^1$ ou les groupes $X^2$ étant respectivement identiques ou différents lorsque m ou n est respectivement égal à 2 ou 3, et les groupes $X^3$ étant respectivement identiques ou différents.

**2.** Composé divinylique selon la revendication 1, dans lequel $R^1$ représente un groupe alkyle ne comportant pas plus de 6 atomes de carbone, un groupe alkoxyalkyle ne comportant pas plus de 6 atomes de carbone, un groupe haloalkyle ne comportant pas plus de 6 atomes de carbone, un groupe alkényle ne comportant pas plus de 6 atomes de carbone, un groupe cyclohexyle, un groupe cyclohexylméthyle, un groupe méthylcyclohexyle, un groupe triméthylcyclohexyle, un groupe furfuryle, un groupe tétrahydrofurfuryle ou un groupe tétrahydropyran-2-yl-méthyle, un groupe benzyle, ou un groupe benzyle substitué avec un groupe alkyle ne comportant pas plus de 4 atomes de carbone; $R^2$ représente n'importe quel groupe $R^1$ défini ci-dessus, un groupe phényle, un groupe phényle substitué avec un atome de chlore ou un groupe alkyle ne comportant pas plus de 4 atomes de carbone; $X^1$ représente un groupe méthyle, un groupe méthoxy ou un groupe éthoxy; $X^2$ représente un atome d'halogène, un groupe alkyle ne comportant pas plus de 6 atomes de carbone, un groupe alkoxy ne comportant pas plus de 6 atomes de carbone, un groupe alkoxyalkoxy ne comportant pas plus de 6 atomes de carbone, un groupe cyclohexyloxy, un groupe benzyloxy, un groupe phénoxy, ou un groupe phénoxy ou benzyloxy substitué avec un groupe alkyle ou alkoxy ne comportant respectivement pas plus de 4 atomes de carbone; m est égal à 0 ou 1, et n est égal à 0,1 ou 2.

**3.** Composé divinylique selon la revendication 2, dans lequel $R^1$ représente un groupe alkyle ne comportant pas plus de 6 atomes de carbone; $R^2$ représente un groupe alkyle ne comportant pas plus de 6 atomes de carbone ou un groupe cyclohexyle; $X^2$ représente un atome d'halogène, un groupe alkyle ne comportant pas plus de 6 atomes de carbone, un groupe alkoxy ne comportant pas plus de 6 atomes de carbone, un groupe alkoxyalkoxy ne comportant pas plus de 6 atomes de carbone, un groupe cyclohexyloxy, un groupe benzyloxy, un groupe phénoxy, ou un groupe phénoxy ou benzyloxy substitué avec un groupe alkyle ou alkoxy ne comportant respectivement pas plus de 4 atomes de carbone; $X^3$ représente un atome de chlore, m est égal à 0, et n est égal à 0,1 ou 2.

**4.** Composé divinylique selon la revendication 3, dans lequel $R^1$ représente un groupe méthyle ou éthyle; $R^2$ représente un groupe méthyle, éthyle, n-propyle ou iso-propyle; $X^2$ représente un groupe méthyle ou un groupe alkoxy ne comportant pas plus de 5 atomes de carbone, et n est égal à 1 ou 2.

**5.** 3,3-bis[2-(p-diméthylaminophényl)-2-(p-méthoxyphényl)éthényl]-4,5,6,7-tétrachlorophtalide.

**6.** 3,3-bis[2-(p-diméthylaminophényl)-2-(p-éthoxyphényl)éthényl]-4,5,6,7-tétrachlorophtalide.

**7.** 3,3-bis[2-(p-diéthylaminophényl)-2-(p-méthoxyphényl)éthényl]-4,5,6,7-tétrachlorophtalide.

**8.** 3,3-bis[2-(p-diméthylaminophényl)-2-(p-propoxyphényl)éthényl]-4,5,6,7-tétrachlorophtalide.

**9.** 3,3-bis[2-(p-diméthylaminophényl)-2-(p-iso-propoxyphényl)éthényl]-4,5,6,7-tétrachlorophtalide.

**10.** 3,3-bis[2-(p-diméthylaminophényl)-2-(p-butoxyphényl)éthényl]-4,5,6,7-tétrachlorophtalide.

**11.** 3,3-bis[2-(p-diméthylaminophényl)-2-(p-iso-butoxyphényl)éthényl]-4,5,6,7-tétrachlorophtalide.

**12.** 3,3-bis[2-(p-méthyléthylaminophényl)-2-(p-méthoxyphényl)éthényl]-4,5,6,7-tétrachlorophtalide.

**13.** 3,3-bis[2-(p-méthylpropylaminophényl)-2-(p-méthoxyphényl)éthényl]-4,5,6,7-tétrachlorophtalide.

**14.** 3,3-bis[2-(p-diméthylaminophényl)-2-(p-méthylphényl)éthényl]-4,5,6,7-tétrachlorophtalide.

**15.** 3,3-bis[2-(p-éthylpropylaminophényl)-2-(p-méthoxyphényl)éthényl]-4,5,6,7-tétrachlorophtalide.

**16.** 3,3-bis[2-(p-méthyl-iso-propylaminophényl)-2-(p-méthoxyphényl)éthényl]-4,5,6,7-tétrachlorophtalide.

**17.** 3,3-bis[2-(p-éthyl-iso-propylaminophényl)-2-(p-méthoxyphényl)éthényl]-4,5,6,7-tétrachlorophtalide.

**18.** 3,3-bis[2-(p-dipropylaminophényl)-2-(p-méthoxyphényl)éthényl]-4,5,6,7-tétrachlorophtalide.

**19.** 3,3-bis[2-(p-méthylbutylaminophényl)-2-(p-méthoxyphényl)éthényl]-4,5,6,7-tétrachlorophtalide.

**20.** 3,3-bis[2-(p-éthylbutylaminophényl)-2-(p-méthoxyphényl)éthényl]-4,5,6,7-tétrachlorophtalide.

**21.** 3,3-bis[2-(p-diéthylaminophényl)-2-(p-éthoxyphényl)éthényl]-4,5,6,7-tétrachlorophtalide.

**22.** 3,3-bis[2-(p-diméthylaminophényl)-2-(m-méthyl-p-méthoxyphényl)éthényl]-4,5,6,7-tétrachlorophtalide.

**23.** 3,3-bis[2-(p-diméthylaminophényl)-2-(p-pentyloxyphényl)éthényl]-4,5,6,7-tétrachlorophtalide.

**24.** 3,3-bis[2-(p-diméthylaminophényl)-2-(p-méthoxyphényl)éthényl]-4,5,6,7-tétrabromophtalide.

**25.** 3,3-bis[2-(p-diméthylaminophényl)-2-(p-isopentyloxyphényl)éthényl]-4,5,6,7-tétrachlorophtalide.

**26.** 3,3-bis[2-(p-diméthylaminophényl)-2-(m,p-diméthoxyphényl)éthényl]-4,5,6,7-tétrachlorophtalide.

**27.** 3,3-bis[2-(p-méthyléthylaminophényl)-2-(péthoxyphényl)éthényl]-4,5,6,7-tétrachlorophtalide.

**28.** 3,3-bis[2-(p-méthylpropylaminophényl)-2-(péthoxyphényl)éthényl]-4,5,6,7-tétrachlorophtalide.

**29.** 3,3-bis[2-(p-diméthylaminophényl)-2-(p-éthylphényl)éthényl]-4,5,6,7-tétrachlorophtalide.

**30.** 3,3-bis[2-(p-diméthylaminophényl)-2-phényléthényl]-4,5,6,7-tétrachlorophtalide.

**31.** 3,3-bis[2-(p-diméthylaminophényl)-2-(p-tertbutoxyphényl)éthényl]-4,5,6,7-tétrachlorophtalide.

**32.** 3,3-bis[2-(p-diméthylaminophényl)-2-(p-sec-butoxyphényl)éthényl]-4,5,6,7-tétrachlorophtalide.

**33.** 3,3-bis[2-(p-méthylcyclohexylaminophényl)-2-(p-méthoxyphényl)éthényl]-4,5,6,7-tétrachlorophtalide.

**34.** 3,3-bis[2-(p-diméthylaminophényl)-2-(p-méthoxyphényl)éthényl]-5,6-dichloro-4,7-dibromophtalide.

**35.** 3,3-bis[2-(p-méthylméthoxyéthylaminophényl)-2-(p-méthoxyphényl)éthényl]-4,5,6,7-tétrachlorophtalide.

**36.** 3,3-bis[2-(p-méthylallylaminophényl)-2-(p-méthylphényl)éthényl]-4,5,6,7-tétrachlorophtalide.

**37.** 3,3-bis[2-(p-diméthylaminophényl)-2-[p-(p-méthoxyphénoxy)phényl]éthényl]-4,5,6,7-tétrachlorophtalide.

**38.** Procédé de préparation d'un composé divinylique de formule (I), défini dans l'une quelconque des revendications précédentes, selon lequel on condense un dérivé d'éthylène de formule (2):

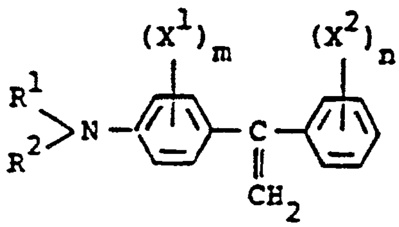
(2)

dans laquelle $R^1$, $R^2$, $X^1$, $X^2$, m et n sont tels que définis dans la revendication 1, avec un dérivé d'acide phtalique de formule (3):

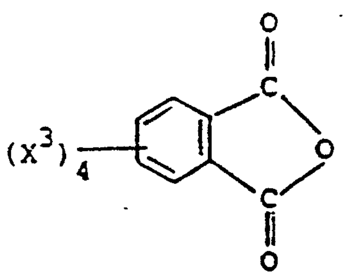
(3)

dans laquelle $X^3$ est tel que défini dans la revendication 1, selon un rapport molaire de 2:1, en présence d'un agent de déshydratation.

**39.** Procédé selon la revendication 38, dans lequel le dérivé d'éthylène de formule (2) est préparé par l'intermédiaire d'une réaction de Grignard entre les composés de formule $CH_3MgX$ et:

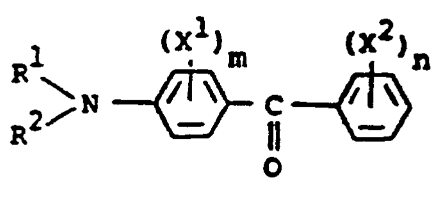

dans lesquelles X représente un atome d'halogène, et $R^1$, $R^2$, $X^1$, $X^2$, m et n, sont tels que définis dans la revendication 38.

**40.** Procédé selon la revendication 38, dans lequel le dérivé d'éthylène de formule (2), est préparé par l'intermédiaire d'une réaction de Grignard entre les composés de formule:

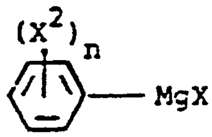

et

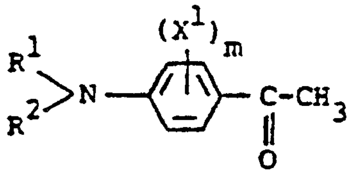

dans lesquelles X représente un atome d'halogène, et $R^1$, $R^2$, $X^1$, $X^2$, m et n sont tels que définis dans la revendication 38.

**41.** Procédé selon la revendication 38, dans lequel le dérivé d'éthylène de formule (2), est préparé par l'intermédiaire d'une réaction de Grignard entre les composés de formule:

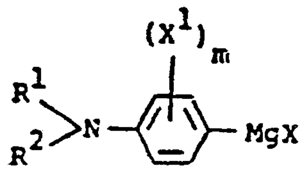

et

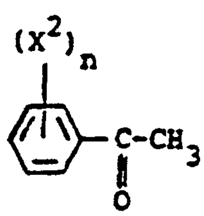

dans lesquelles X représente un atome d'halogène, et $R^1$, $R^2$, $X^1$, $X^2$, m et n sont tels que définis dans la revendication 38.

**42.** Matériau d'enregistrement comprenant, en tant qu'agent chromogène, un composé divinylique de formule (I) selon l'une quelconque des revendications 1 à 37, ou préparé en mettant en oeuvre un procédé selon l'une quelconque des revendications 38 à 41.

**43.** Matériau d'enregistrement selon la revendication 42, consistant en un papier d'enregistrement sensible à la pression, dans lequel le composé divinylique est encapsulé sous la forme d'une solution dans des microcapsules présentes sur une face d'une feuille de papier, en contact avec une face d'une autre feuille de papier, revêtue d'un révélateur.

**44.** Marériau d'enregistrement selon la revendication 42, consistant en un papier d'enregistrement sensible à la pression, une face d'une feuille de papier étant revêtue d'un mélange comprenant le composé divinylique, un liant et un révélateur.

45. Utilisation d'un agent chromogène à base d'un composé divinylique de formule (I) selon l'une quelconque des revendications 1 à 37, ou préparé en mettant en oeuvre un procédé selon l'une quelconque des revendications 38 à 41.

46. Matériau d'enregistrement selon l'une quelconque des revendications 42 à 44, comprenant au moins deux desdits composés divinyliques.

47. Matériau d'enregistrement selon l'une quelconque des revendications 42 à 44 ou 46, comprenant en outre un agent chromogène supplémentaire ayant la structure de base du 3,3-bis-(aminophényl)-6-aminophtalide, du 3,3-bis(indolyl)-phtalide, du 3-aminofluoranne, de l'aminobenzofluoranne, du 2,6-diaminofluoranne, du 2,6-diaminofluoranne, du 2,6-diamino-3-méthylfluoranne, du spiropyranne, de la phénothiazine, de la phénoxazine, de la leucoauramine, du diarylcarbazolylméthane, du 3-indolyl-3-(aminophényl)-azaphtalide, du triaminofluorènephtalide ou du tétraaminodivinylphtalide.

## Patentansprüche

1. Divinylverbindung, dargestellt durch die Formel (I):

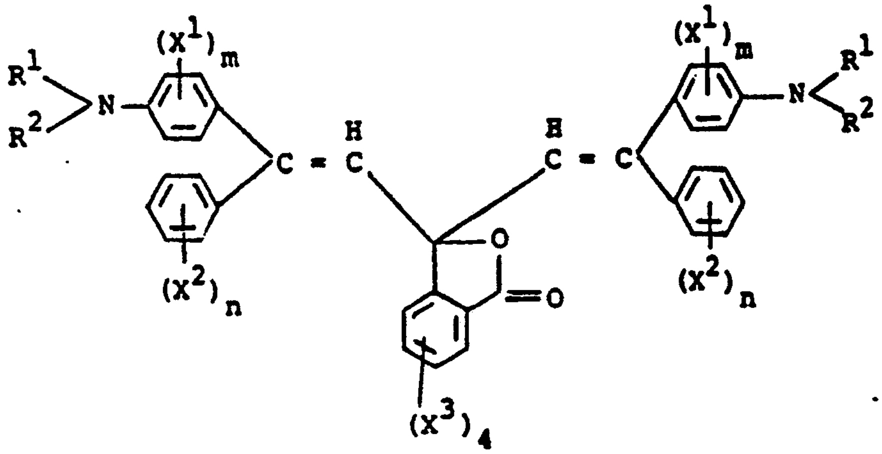

(I)

wobei $x^1$ eine Alkylgruppe, eine Alkoxygruppe oder ein Halogenatom darstellt; $x^2$ eine Alkylgruppe, eine Alkoxygruppe, ein Halogenatom oder $R^3O$- darstellt; $x^3$ ein Halogenatom dargestellt; $R^1$ ein Wasserstoffatom, eine Alkylgruppe, eine Alkoxyalkylgruppe, eine Haloalkylgruppe, eine Alkylengruppe, eine Aralkylgruppe, eine Cyclohexylmethylgruppe, eine Methylcyclohexylgruppe, eine Trimethylcyclohexylgruppe, eine Furfurylgruppe, eine Tetrahydrofurfurylgruppe oder eine Tetrahydrohydropyran-2-yl-methylgruppe darstellt; $R^2$ eine Gruppe, die unter $R^1$ definiert ist, eine Cycloalkylgruppe, eine Phenylgruppe, eine Benzylgruppe oder eine Phenyl- oder eine Benzylgruppe, substituiert durch Chloratome, Bromatome oder Alkylgruppen, die nicht mehr als vier Kohlenstoffatome haben, oder durch eine Alkoxygruppe, die nicht mehr als vier Kohlenstoffatome hat, darstellt; $R^3$ eine Arylgruppe, eine Cycloalkylgruppe, eine Alkoxyalkylgruppe, eine Alkylengruppe, eine Haloalkylgruppe oder eine Arakylgruppe darstellt; und m und n je 0, 1, 2 oder 3 darstellen; wobei jedes $x^1$ oder jedes $x^2$ gleich oder verschieden ist, wenn m oder n jeweils 2 oder 3 ist, und jedes $x^3$ gleich oder verschieden ist.

2. Divinylverbindung nach Anspruch 1, wobei $R^1$ eine Alkylgruppe mit nicht mehr als 6 Kohlenstoffatomen, eine Alkoxyalkylgruppe mit nicht mehr als 6 Kohlenstoffatomen, eine Haloalkylgruppe mit nicht mehr als 6 Kohlenstoffatomen, eine Alkenylgruppe mit nicht mehr als 6 Kohlenstoffatomen, eine Cyclohexylgruppe, eine Cyclohexylmethylgruppe, eine Methylcyclohexylgruppe, eine Trimethylcyclohexylgruppe, eine Furfurylgruppe, eine Tetrahydrofurfurylgruppe, eine Tetrahydropyran-2-yl-methylgruppe, eine Benzylgruppe oder eine Benzylgruppe, die mit einer Alkylgruppe mit nicht mehr als 4 Kohlenstoffatomen substituiert ist, darstellt; $R^2$ eine Gruppe, die unter $R^1$ definiert ist, eine Phenylgruppe, eine Phenylgruppe, die mit einem Chloratom oder einer Alkylgruppe mit nicht mehr als 4 Kohlenstoffatomen substituiert ist, darstellt; $x^1$ eine Methylgruppe, eine Methoxygruppe oder eine Ethoxygruppe darstellt; $x^2$ ein Halogenatom, eine Alkylgruppe mit nicht mehr als 6 Kohlenstoffatomen, eine Alkoxygruppe mit nicht mehr als 6 Kohlenstoffatomen, eine Alkoxyalkoxygruppe mit nicht mehr als 6 Kohlenstoffatomen, eine

Cyclohexyloxygruppe, eine Benzyloxygruppe, eine Phenoxygruppe oder eine Phenoxy-oder eine Benzyloxygruppe, die mit einer Alkyl- oder einer Alkoxygruppe substituiert ist, die beide nicht mehr als 4 Kohlenstoffatome haben, darstellt; m 0 oder 1 ist und n 0, 1 oder 2 ist.

**3.** Divinylverbindung nach Anspruch 2, wobei $R^1$ eine Alkylgruppe mit nicht mehr als 6 Kohlenstoffatomen darstellt; $R^2$ eine Alkylgruppe mit nicht mehr als 6 Kohlenstoffatomen oder Cyclohexylgruppe darstellt; $x^2$ ein Halogenatom, eine Alkylgruppe mit nicht mehr als 6 Kohlenstoffatomen, eine Alkoxygruppe mit nicht mehr als 6 Kohlenstoffen, eine Alkoxyalkoxygruppe mit nicht mehr als 6 Kohlenstoffatomen, eine Cyclohexyloxygruppe, eine Phenoxygruppe, eine Benzyloxygruppe oder Phenoxy- oder Benzyloxygruppe, die mit einer Alkyl- oder Alkoxygruppe substituiert ist, die beide nicht mehr als 4 Kohlenstoffatome haben, darstellt; $x^3$ ein Chloratom darstellt, m 0 ist und n 0, 1 oder 2 ist.

**4.** Divinylverbindung nach Anspruch 3, wobei $R^1$ eine Methylgruppe oder eine Ethylgruppe darstellt; $R^2$ eine Methylgruppe, eine Ethylgruppe, eine n-Propylgruppe oder eine Isopropylgruppe darstellt; $x^2$ eine Methylgruppe, eine Alkoxygruppe mit nicht mehr als 5 Kohlenstoffatomen darstellt und n 1 oder 2 ist.

**5.** 3,3-Bis[2-(p-dimethylaminophenyl)-2-(p-methoxyphenyl)ethenyl]-4,5,6,7-tetrachlorphthalid.

**6.** 3,3-Bis[2-(p-dimethylaminophenyl)-2-(p-ethoxyphenyl)ethenyl]-4,5,6,7-tetrachlorphthalid.

**7.** 3,3-Bis[2-(p-diethylaminophenyl)-2-(methoxyphenyl)ethenyl]-4,5,6,7-tetrachlorphthalid.

**8.** 3,3-Bis[2-(p-dimethylaminophenyl)-2-(p-propoxyphenyl)ethenyl]-4,5,6,7-tetrachlorphthalid.

**9.** 3,3-Bis[2-(p-dimethylaminophenyl)-2-(p-iso-propoxyphenyl)ethenyl]-4,5,6,7-tetrachlorphthalid.

**10.** 3,3-Bis[2-(p-dimethylaminophenyl)-2-(p-butoxyphenyl)ethenyl]-4,5,6,7-tetrachlorphthalid.

**11.** 3,3-Bis[2-(p-dimethylaminophenyl)-2-(p-iso-butoxyphenyl)ethenyl]-4,5,6,7-tetrachlorphthalid.

**12.** 3,3-Bis[2-(p-methylethylaminophenyl)-2-(p-methoxyphenyl)ethenyl]-4,5,6,7-tetrachlorphthalid.

**13.** 3,3-Bis[2-(p-methylpropylaminophenyl)-2-(p-methoxyphenyl)ethenyl]-4,5,6,7-tetrachlorphthalid.

**14.** 3,3-Bis[2-(p-dimethylaminophenyl)-2-(p-methylphenyl)ethenyl]-4,5,6,7-tetrachlorphthalid.

**15.** 3,3-Bis[2-(p-ethylpropylaminophenyl)-2-(p-methoxyphenyl)ethenyl]-4,5,6,7-tetrachlorphthalid.

**16.** 3,3-Bis[2-(p-methyl-iso-propylaminophenyl)-2-(p-methoxyphenyl)ethenyl]-4,5,6,7-tetrachlorphthalid.

**17.** 3,3-Bis[2-(p-ethyl-iso-propylaminophenyl)-2-(p-methoxyphenyl)ethenyl]-4,5,6,7-tetrachlorphthalid.

**18.** 3,3-Bis[2-(p-dipropylaminophenyl)-2-(p-methoxyphenyl)ethenyl]-4,5,6,7-tetrachlorphthalid.

**19.** 3,3-Bis[2-(p-methylbutylaminophenyl)-2-(p-methoxyphenyl)ethenyl]-4,5,6,7-tetrachlorphthalid.

**20.** 3,3-Bis[2-(p-ethylbutylaminophenyl)-2-(p-methoxyphenyl)ethenyl]-4,5,6,7-tetrachlorphthalid.

**21.** 3,3-Bis[2-(p-diethylaminophenyl)-2-(p-ethoxyphenyl)ethenyl]-4,5,6,7-tetrachlorphthalid.

**22.** 3,3-Bis[2-(p-dimethylaminophenyl)-2-(m-methyl-p-methoxyphenyl)ethenyl]-4,5,6,7-tetrachlorphthalid.

**23.** 3,3-Bis[2-(p-dimethylaminophenyl)-2-(p-pentyloxyphenyl)ethenyl]-4,5,6,7-tetrachlorphthalid.

**24.** 3,3-Bis[2-(p-dimethylaminophenyl)-2-(p-methoxyphenyl)ethenyl]-4,5,6,7-tetrabromphthalid.

**25.** 3,3-Bis[2-(p-dimethylaminophenyl)-2-(p-iso-pentyloxyphenyl)ethenyl]-4,5,6,7-tetrachlorphthalid.

**26.** 3,3-Bis[2-(p-dimethylaminophenyl)-2-(m,p-dimethoxyphenyl)ethenyl]-4,5,6,7-tetrachlorphthalid.

**27.** 3,3-Bis[2-(p-methylethylaminophenyl)-2-(p-ethoxyphenyl)ethenyl]-4,5,6,7-tetrachlorphthalid.

**28.** 3,3-Bis[2-(p-methylpropylaminophenyl)-2-(p-ethoxyphenyl)ethenyl]-4,5,6,7-tetrachlorphthalid.

**29.** 3,3-Bis[2-(p-dimethylaminophenyl)-2-(p-ethylphenyl)ethenyl]-4,5,6,7-tetrachlorphthalid.

**30.** 3,3-Bis[2-(p-dimethylaminophenyl)-2-phenylethenyl]-4,5,6,7-tetrachlorphthalid.

**31.** 3,3-Bis[2-(p-dimethylaminophenyl)-2-(p-tert.-butoxyphenyl)ethenyl]-4,5,6,7-tetrachlorphthalid.

**32.** 3,3-Bis[2-(p-dimethylaminophenyl)-2-(p-sec.-butoxyphenyl)ethenyl]-4,5,6,7-tetrachlorphthalid.

**33.** 3,3-Bis[2-(p-methylcyclohexylaminophenyl)-2-(p-methoxyphenyl)ethenyl]-4,5,6,7-tetrachlorphthalid.

**34.** 3,3-Bis[2-(p-dimethylaminophenyl)-2-(p-methoxyphenyl)ethenyl]-5,6-dichlor-4,7-dibromphthalid.

**35.** 3,3-Bis[2-(p-methylmethoxyethylaminophenyl)-2-(p-methoxyphenyl)ethenyl]-4,5,6,7-tetrachlorphthalid.

**36.** 3,3-Bis[2-(p-methylallylaminophenyl)-2-(p-methylphenyl)ethenyl]-4,5,6,7-tetrachlorphthalid.

**37.** 3,3-Bis 2-(p-dimethylaminophenyl)-2-[p-(p-methoxyphenoxy)phenyl]ethenyl}-4,5,6,7-tetrachlorphthalid.

**38.** Verfahren zur Herstellung einer Divinylverbindung, dargestellt durch die Formel (I) wie in einem der vorhergehenden Ansprüche definiert, bei dem das Verfahren die Kondensation eines Ethylenderivats der Formel (2):

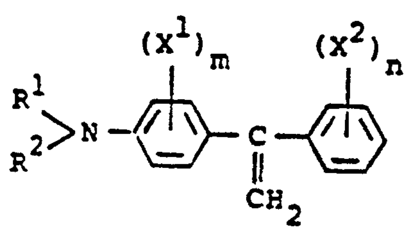
(2)

wobei $R^1$, $R^2$, $x^1$, $x^2$, m und n wie in Anspruch 1 definiert sind, mit einem Phthalsäurederivat der Formel (3)

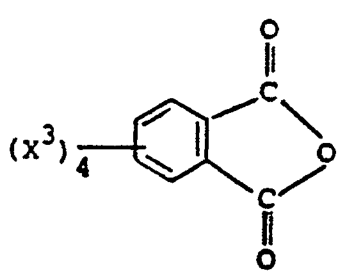
(3)

wobei $x^3$ wie in Anspruch 1 definiert ist, in einem molaren Verhältnis von 2:1 und in der Gegenwart eines Dehydrierungsmittels beinhaltet.

**39.** Verfahren nach Anspruch 38, wobei das Ethylenderivat der Formel (2) durch Grignard-Reaktion zwischen den Komponenten $CH_3MgX$ und

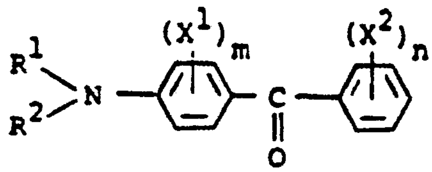

hergestellt wurde, wobei X ein Halogenatom darstellt und $R^1$, $R^2$, $x^1$, $x^2$, m und n wie in Anspruch 38 definiert sind.

**40.** Verfahren nach Anspruch 38, bei dem das Ethylenderivat der Formel (2) mittels Grignard-Reaktion zwischen den Komponenten

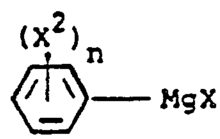

und

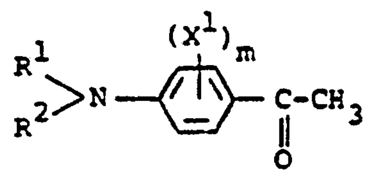

hergestellt wurde, wobei X ein Halogenatom darstellt und $R^1$, $R^2$, $x^1$, $x^2$, m und n wie in Anspruch 38 definiert sind.

**41.** Verfahren nach Anspruch 38, bei dem das Ethylenderivat der Formel (2) mittels Grignard-Reaktion und zwischen den Komponenten

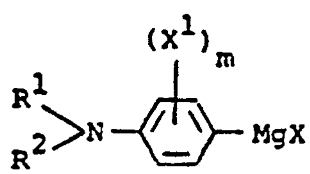

und

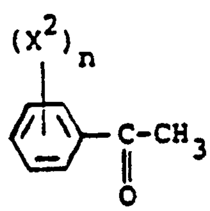

hergestellt wurde, wobei X ein Halogenatom darstellt und $R^1$, $R^2$, $x^1$, $x^2$, m und n wie in Anspruch 38 definiert sind.

**42.** Aufzeichnungsmaterial, das als chromogenes Agens eine Divinylverbindung enthält, die durch die Formel (I) dargestellt ist, wie in einem der Ansprüche 1 bis 37 beansprucht oder die durch ein Verfahren hergestellt wurde, wie in einem der Ansprüche 38 bis 41 beansprucht.

**43.** Aufzeichnungsmaterial nach Anspruch 42, das ein drucksensitives Aufzeichnungspapier ist, wobei die Divinylverbindung in einer Lösung innerhalb von Mikrokapseln eingekapselt ist, die auf einer Oberseite des Papiers vorhanden sind, und eine gegenüberliegende Seite eines weiteren Papiers mit einem Entwickler überzogen ist.

**44.** Aufzeichnungsmaterial nach Anspruch 42, das ein hitzesensitives Aufzeichnungspapier ist, wobei eine Mischung, die die Divinylverbindung, ein Bindemittel und einen Entwickler aufweist, eine Seite des Papiers beschichtet.

**45.** Verwendung als ein chromogenes Agens einer Divinylverbindung, die durch die Formel (I) dargestellt ist, wie in einem der Ansprüche 1 bis 38 beansprucht, oder die durch ein Verfahren hergestellt wurde, wie in einem der Ansprüche 38 bis 41 beansprucht.

**46.** Aufzeichnungsmaterial nach einem der Ansprüche 42 bis 44, das wenigstens 2 Divinylverbindungen enthält.

**47.** Aufzeichnungsmaterial nach einem der Ansprüche 42 bis 44 oder 46, das des weiteren ein zusätzliches chromogenes Agens mit dem fundamentalen Grundgerüst von 3,3-Bis-(aminophenyl)-6-aminophthalid, 3,3-Bis(indolyl)-phthalid, 3-Aminofluoran, Aminobenzofluoran, 2,6-Diaminofluoran, 2,6-Diamino-3-methylfluoran, Spiropyran, Phenothiazin, Phenoxazin, Leucoauramin, Diarylcarbazolylmethan, 3- Indolyl-3-(aminophenyl)-azaphthalid, Triamino-fluorenphthalid oder Tetraaminodivinylphthalid enthält.

Fig. 1

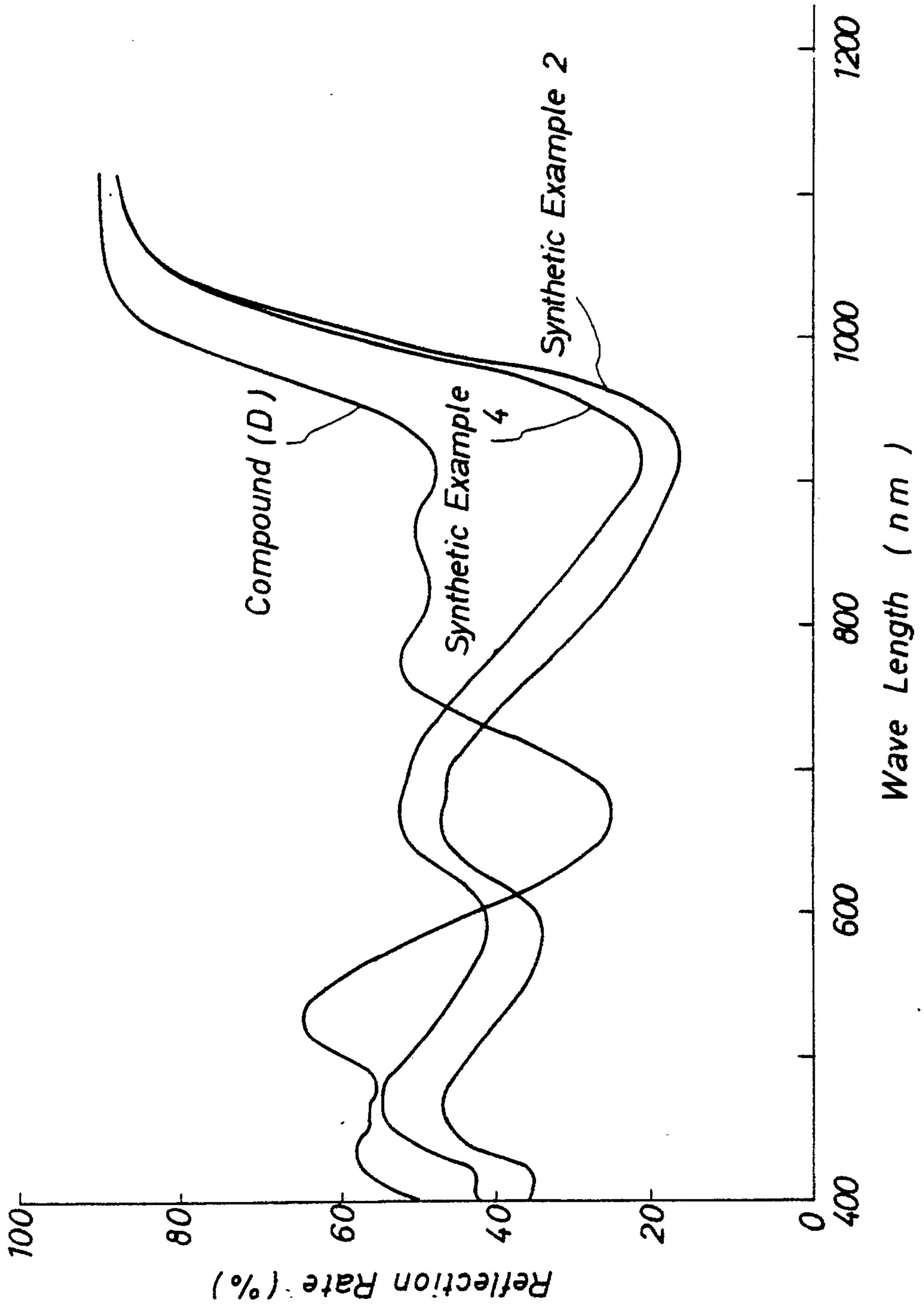
Compound (D)
Synthetic Example 4
Synthetic Example 2
Reflection Rate (%)
Wave Length (nm)
0
20
40
60
80
100
400
600
800
1000
1200

Fig.2

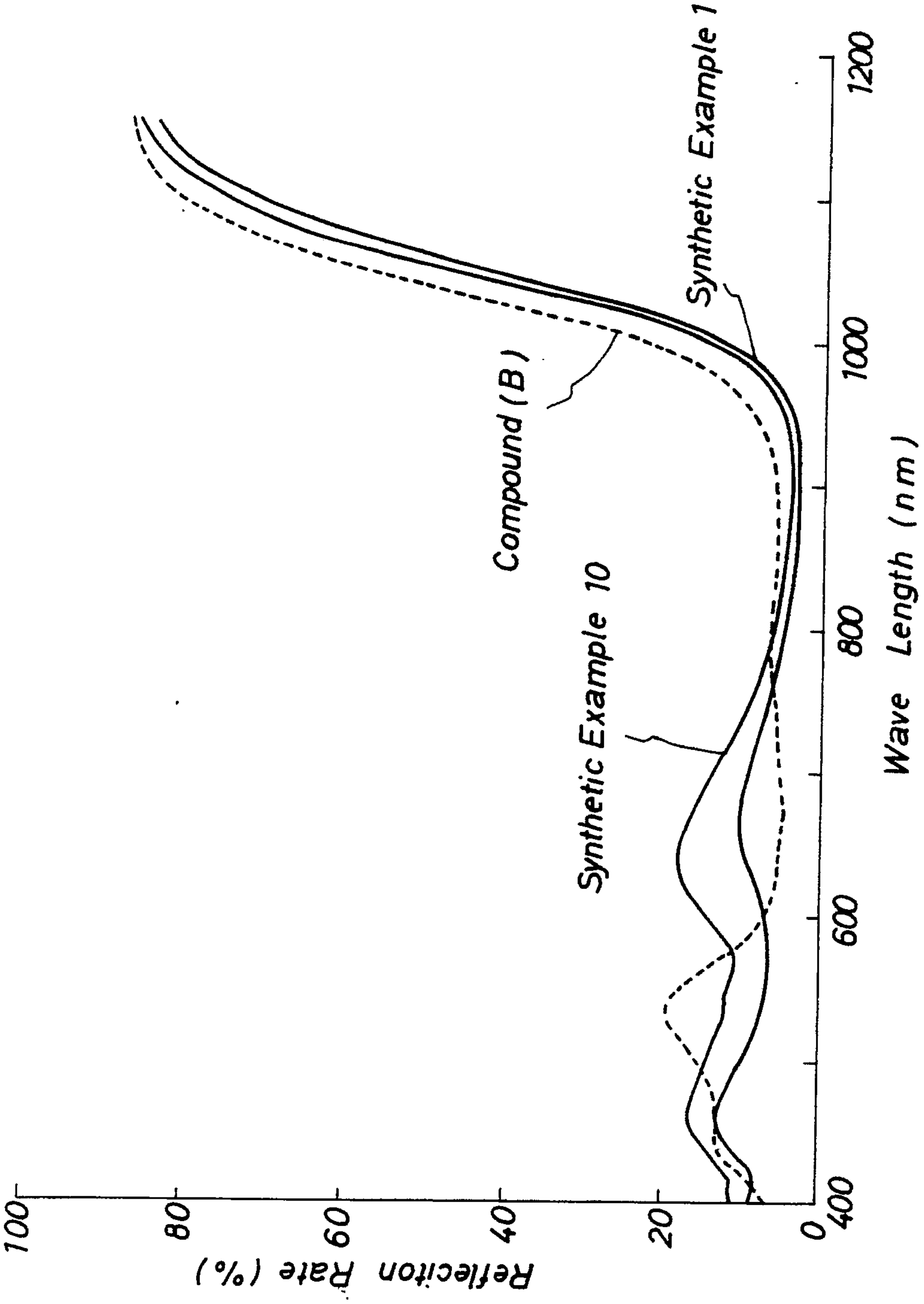
Reflection Rate (%)
100
80
60
40
20
0
400
600
800
1000
1200
Wave Length (nm)
Synthetic Example 1
Compound (B)
Synthetic Example 10

Fig.3

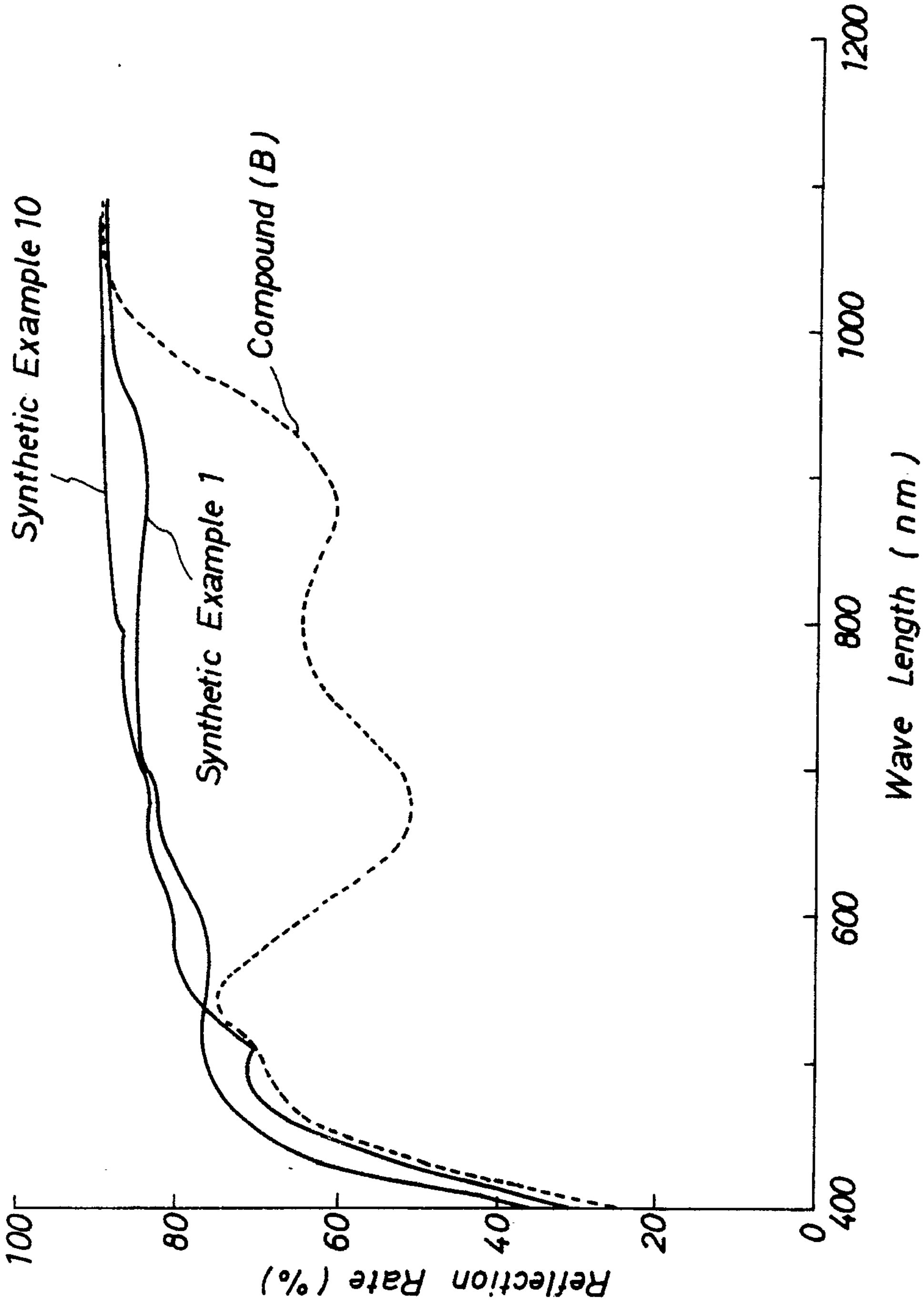
Synthetic Example 10
Synthetic Example 1
Compound (B)
Reflection Rate (%)
Wave Length (nm)
0
20
40
60
80
100
400
600
800
1000
1200